# EUROPEAN PATENT APPLICATION

(11) **EP 1 598 368 A1**
(43) Date of publication of application: **23.11.2005**
(21) Application number: 04703509.2
(22) Date of filing: 20.01.2004
(51) Int. Cl.: C07K 16/18, C12N 15/13, C12P 21/08, A61K 39/395, A61P 7/02

(54) **ANTI-PCI NEUTRALIZING ANTIBODY**

(30) Priority: 20.01.2003 JP 2003011529
(71) Applicant: CHUGAI SEIYAKU KABUSHIKI KAISHA, Tokyo, 115-8543 (JP)
(72) Inventor: KOGA, Takaki c/o CHUGAI SEIYAKU KABUSHIKI KAISHA, Gotenba-shi, Shizuoka 4128513 (JP); KIMURA, Naoki c/o CHUGAI SEIYAKU KABUSHIKI KAISHA, Niihari-gun, Ibaraki 30041 (JP); YOSHINO, Takeshi c/o CHUGAI SEIYAKU KABUSHIKI K., Niihari-gun, Ibaraki 30041 (JP); ONO, Koichiro c/o CHUGAI SEIYAKU KABUSHIKI KAISHA, Niihari-gun, Ibaraki 30041 (JP)
(74) Representative: Vossius & Partner
(86) International application number: PCT/JP2004/000429
(87) International publication number: WO 2004/065418

(57) **Abstract**

The present invention provides anti-PCI antibodies having Protein C inhibitor (PCI)-neutralizing activity, and the uses thereof. Through the generation and screening of anti-PCI antibodies, the inventors successfully isolated anti-PCI antibodies which inhibit PCI's inhibitory effect on the production and activity of activated Protein C (aPC). The antibodies of the present invention suppress PCI's inhibitory effect on aPC production and/or the aPC inactivation by PCI, and thus can be used to maintain aPC activity and sustain the effects of aPC physiological activities, such as suppression of the activation of blood coagulation system and anti-inflammatory functions. The present invention also provides uses of the antibodies of the present invention in treating diseases such as thrombosis and sepsis using aPC. In treatments by aPC administration, the therapeutic effect of aPC can be sustained by administering an antibody of the present invention. The antibodies of the present invention can be used in the treatment and prevention of diseases such as thrombosis and sepsis.

## Description

### Technical Field

The present invention relates to neutralizing antibodies against Protein C Inhibitor (PCI).

### Background Art

Venous thrombosis frequently develops after major abdominal surgery or lower limb arthroplasty. The main treatment currently practiced involves prevention using low-molecular-weight heparin and warfarin. However, low-molecular-weight heparin requires daily subcutaneous administration. Warfarin can be administered orally; however, its interaction with other drugs has become a problem because of the exceedingly high protein-binding rate. In addition, a bleeding tendency is seen with both drugs.

Disseminated intravascular coagulation (DIC) is a clinical condition in which intravascular blood coagulation progresses throughout the body's blood vessels, resulting in organ failures due to inadequate circulation and hemorrhagic symptoms due to excessive consumption of blood coagulation factors. DIC is caused by leukemia, solid tumors, infectious diseases, obstetric diseases, and the like. Heparin (intravenous or subcutaneous administration) has been widely used to treat DIC, anticipating its anticoagulant activity. However, the drug is disadvantageous in that it promotes hemorrhage and does not give sufficient effects at low antithrombin III concentrations. Synthetic protease inhibitors may also be prescribed, but their effectiveness is not always clear.

In sepsis, cellular components of infecting bacteria may induce a coagulation reaction and cause DIC. However, there are very few effective drugs for treating sepsis, and only activated Protein C (aPC) has been approved in the U.S. so far.

Anticoagulants such as heparin and synthetic antithrombin agents are also prescribed for other clinical conditions associated with the blood coagulation system, for example, coronary artery syndrome and peripheral circulatory failure. However, the required daily administration of such agents impairs patients' quality of life (QOL). Therefore, antithrombotic drugs which have long-lasting effects (a few weeks) and do not have a tendency to cause bleeding can prevent thrombosis and improve patients' QOL.

A blood clot (thrombus) is formed by the activation of platelets and the blood coagulation system. It is believed that platelets chiefly contribute to the formation of an arterial thrombus, while the coagulation system mainly contributes to the formation of a venous thrombus. Activation of the blood coagulation system brings about thrombin formation, which leads to the production of fibrin, a major component in the thrombus network. Meanwhile, thrombin alters its own properties upon binding to thrombomodulin on the surface of vascular endothelia, thus activating Protein C (PC). The activated PC (aPC) uses protein S as a coenzyme to inactivate Factors Va and VIIIa, thereby suppressing the coagulation system. Furthermore, aPC comprises the activity of suppressing fibrinolysis-inhibiting substances, such as PAI-1 (plasminogen activator inhibitor-1) and TAFI (thrombin activatable fibrinolysis inhibitor), and thus enhances the fibrinolysis system. PC and aPC are thus presumed to play important roles in a negative feedback mechanism for the activated blood coagulation system. Indeed, the fact that both congenital PC deficiency and aPC resistance (due to factor Va mutations) can be causative factors in thrombosis reinforces the importance of aPC's role in thrombosis. Recent studies suggest that aPC acts on vascular endothelia and has an anti-inflammatory activity (J. Biol. Chem. 2001, 276:11199-11203). It has also been reported that aPC reduces mortality rate in a sepsis model (J. Clin. Invest. 1987, 79:918-925) and that this effect cannot be explained by its anticoagulant activity alone (Blood 1991, 78:364-368). These findings suggest that aPC is effective for treating and preventing thrombosis and sepsis.

However, since aPC has an extremely short half-life of 20-30 minutes in blood, continuous intravenous administration or long periods of repetitive administration is required when it is used as a drug. This is disadvantageous in terms of healthcare economics and patients' quality of life. aPC's short half-life can be attributed to its irreversible inactivation by *in vivo* inactivators such as PCI and α1-antitrypsin (AAT). Among these inactivators, PCI is believed to play a physiologically important role (Fibrinolysis & Proteolysis 2000, 14:133-145). In fact, elevated blood concentration of the aPC/PCI complex has been reported in clinical conditions such as acute coronary syndrome, DIC, and deep vein thrombosis (Blood Coagul. Fibrinolysis 2001, 12:503-510; Am. J. Hematol. 2000, 65:35-40; Thromb. Haemost. 2001, 86:1400-1408).

PCI irreversibly inhibits aPC enzymatic activity by forming an acyl enzyme complex with aPC (J. Biochem. 1984, 95:187-195). In addition, PCI inhibits thrombin/thrombomodulin (Thr/TM) complex, which is an aPC producing enzyme, and thereby suppresses aPC production (Blood 1998, 91:1542-1547). In other words, PCI suppresses aPC function by inhibiting both its production and activity. Consequently, activity of endogenously produced aPC or exogenously administered aPC can be enhanced for effective anticoagulation through the inhibition of PCI activity.

### Disclosure of the Invention

An objective of the present invention is to provide anti-PCI antibodies having neutralizing activity towards PCI, which inhibits the production and enzymatic activity of aPC, and uses thereof.

PCI (Suzuki, K. et al., J. Biol. Chem. 1983, 258:163-168; Suzuki, K., Fibrinolysis Proteolysis 2000, 14: 133-145) is a soluble protein in blood having a half-life of 23 hours. Accordingly, if PCI-neutralizing antibodies are prepared and sufficient amounts are administered, these antibodies can serve as long-lasting anticoagulants.

As described above, PCI has (1) the activity of inhibiting aPC production (namely, a PC activation inhibitory effect) by the Thr/TM complex, and (2) the activity of inhibiting aPC activity. The present inventors therefore prepared hybridomas producing monoclonal antibodies against PCI and screened for antibodies with the activity to inhibit either of the two PCI activities described above. As a result, the inventors successfully isolated antibodies with inhibitory activity against (1) or (2), with some of the isolated antibodies inhibiting both activities. The antibodies of the present invention suppress the blood coagulation system by enhancing aPC activity, and are thus highly useful for thrombosis treatment and prevention. Further, when used in combination with aPC in the treatment of sepsis and such using aPC, the antibodies of the present invention can be used as pharmaceutical agents that enhance aPC actions by suppressing aPC inactivation in blood.

Specifically, the present invention relates to anti-PCI antibodies having a neutralizing activity towards PCI, which inhibits aPC production and enzymatic activity, and to uses thereof. More specifically, the present invention relates to each of the inventions set forth in the claims. The present invention also relates to inventions comprising a desired combination of one or more (or all) inventions set forth in the claims, in particular, to inventions comprising a desired combination of one or more (or all) inventions set forth in claims (dependent claims) citing the same independent claim(s) (claim(s) relating to inventions not encompassed by inventions recited in other claims). An invention set forth in an independent claim is also intended to include any combinations of the inventions set forth in its dependent claims. Specifically, the present invention includes:
[1] an anti-PCI antibody, having at least any one of: (a) activity to inhibit an inhibitory effect of Protein C inhibitor (PCI) on activated Protein C (aPC) activity, or (b) activity to inhibit an inhibitory effect of Protein C inhibitor (PCI) on the production of activated Protein C (aPC) by thrombin /thrombomodulin (Thr/TM) complex;
[2] an anti-PCI antibody, having both (a) activity to inhibit an inhibitory effect of Protein C inhibitor (PCI) on activated Protein C (aPC) activity, and (b) activity to inhibit an inhibitory effect of Protein C inhibitor (PCI) on the production of activated Protein C (aPC) by thrombin /thrombomodulin (Thr/TM) complex;
[3] the antibody of [1] or [2], wherein the antibody competes for the antibody-binding site with an antibody comprising a variable region of an antibody selected from the group consisting of PC19G8, PC23A7, PC23D8, PC30G1, PC31E2, PC31F1, and PC39C6;
[4] the antibody of any one of [1] to [3], wherein the antibody comprises complementarity determining regions consisting of the amino acid sequences of any one of (a) to (f), or complementarity determining regions functionally equivalent thereto:
   (a) the amino acid sequences of SEQ ID NOs: 49, 50, and 51;
   (b) the amino acid sequences of SEQ ID NOs: 55, 56, and 57;
   (c) the amino acid sequences of SEQ ID NOs: 52, 53, and 54;
   (d) the amino acid sequences of SEQ ID NOs: 58, 59, and 60;
   (e) the amino acid sequence of SEQ ID NOs: 25, 31, and 36; and
   (f) the amino acid sequences of SEQ ID NOs: 41, 45, and 48;
[5] the antibody of any one of [1] to [4], wherein the antibody is selected from the group consisting of human antibodies, humanized antibodies, chimeric antibodies, antibody fragments, single-chain antibodies, and diabodies;
[6] a composition comprising the antibody of any one of [1] to [5] and a pharmaceutically acceptable carrier;
[7] the composition of [6], further comprising Protein C and/or activated Protein C;
[8] the composition of [6] or [7], wherein the composition is a pharmaceutical composition used to prevent or treat a disease that has developed and/or advanced due to a decrease or deficiency of activated Protein C activity;
[9] the composition of [8], wherein the disease is caused by hypercoagulation and/or a hyperinflammatory reaction;
[10] the composition of [9], wherein the disease caused by hypercoagulation and/or a hyperinflammatory reaction is selected from the group consisting of sepsis, disseminated intravascular coagulation syndrome, arterial thrombosis, and venous thrombosis;
[11] a method for preventing or treating a disease that has developed and/or advanced due to a decrease or deficiency of activated Protein C activity, wherein the method comprises the step of administering (a) Protein C and/or activated Protein C, and (b) the antibody of any one of [1] to [5];
[12] a method for preventing or treating a disease that has developed and/or advanced due to a decrease or deficiency of activated Protein C activity, wherein the method comprises the step of administering the antibody of any one of [1] to [5];
[13] a kit used to prevent or treat a disease that has developed and/or advanced due to a decrease or deficiency of activated Protein C activity, wherein the kit comprises (a) the antibody of any one of [1] to [5], and (b) Protein C, activated Protein C, or both; and
[14] a kit used to prevent or treat a disease that has developed and/or advanced due to a decrease or deficiency of activated Protein C activity, wherein the kit comprises (a) Protein C, activated Protein C, and the antibody of [1] to [5]; (b) a recording medium comprising a description on the combined use of (i) a therapeutically effective amount of Protein C and/or activated Protein C and (ii) the antibody of [1] or [2], or a link to the description.

The present invention provides anti-PCI antibodies having neutralizing activity towards PCI, which inhibits aPC production and/or its enzymatic activity, and uses thereof. PCI has inhibitory activities against (1) aPC production (namely, PC activation) by Thr/TM complex and (2) aPC activity. The antibodies of the present invention have significant activity in inhibiting either one of the (1) and (2) PCI activities, and more preferably both of activities (1) and (2). Inhibition of these activities can be detected, for example, by the methods described in the Examples, or by other methods. Specifically, PCI's inhibitory effect on aPC activity can be determined by the following procedure: incubate PCI with an anti-PCI antibody, add the mixture to an aPC solution for incubation, and determine the aPC activity. The percentage of the PCI's inhibitory effect on aPC activity inhibited by an anti-PCI antibody can be determined based on the aPC activity level when PCI is absent (aPC activity without PCI inhibition) and when the anti-PCI antibody is absent (aPC activity when inhibited by PCI). When the incubation of PCI and an antibody results in a lower degree of PCI inhibition against aPC activity than in the absence of the antibody, the antibody is judged to have inhibitory activity against the PCI inhibitory effect on aPC activity. An example of the aPC activity is anticoagulant activity, which can be quantified by, for example, measuring the activated partial thromboplastin time (APTT) using known methods. Alternatively, the aPC activity can be assayed using low-molecular-weight compounds, such as the pyroGlu-Pro-Arg-pNA-HCl (S-2366) chromogenic substrate (see Examples).

PCI's inhibitory effect on aPC production by Thr/TM complex can be determined, for example, by the following procedure: incubate PCI, an anti-PCI antibody, thrombin (Thr), and thrombomodulin (TM) together, add PC to the mixture, perform an aPC formation reaction, and then assay aPC activity to determine the amount of aPC produced. The percentage at which the "PCI's inhibitory effect on aPC production by Thr/TM complex" is inhibited by an anti-PCI antibody, can be determined based on the aPC activity level when PCI is absent (aPC production without PCI inhibition) and when anti-PCI antibody is absent (aPC production when inhibited by PCI). When the addition of an antibody results in a lower degree of PCI inhibition against aPC production as compared to in the absence of the antibody, the antibody is judged to have the activity to inhibit "PCI's inhibitory effect on aPC production by Thr/TM complex." aPC activity can be quantitated using the same method described above.

The present invention also provides methods of screening for anti-PCI antibodies having PCI-neutralizing activity, which comprise the steps of: (i) determining the PCI inhibitory effect on aPC activity when it is bound or not bound by an anti-PCI antibody; and (ii) selecting an antibody that suppresses PCI's inhibitory effect in antibody-bound PCI as compared with antibody-free PCI. The present invention also provides methods of screening for anti-PCI antibodies having PCI-neutralizing activity, which comprise the steps of: (i) determining the PCI inhibitory effect on aPC production by Thr/TM complex when PCI is bound or not bound by an anti-PCI antibody; and (ii) selecting an antibody that suppresses PCI's inhibitory effect in antibody-bound PCI as compared with antibody-free PCI. The present invention also provides antibodies that can be obtained by these screening methods. The antibodies of the present invention can increase *in vivo* aPC production and/or extend aPC life time, and thus enhance aPC activity, by preventing the inhibition of aPC production and/or activity in blood.

The anti-PCI antibodies of the present invention may be monoclonal antibodies (including full-length monoclonal antibodies) or polyclonal antibodies, or mutants of such antibodies. Monoclonal antibodies are preferred because they are stably produced as homogeneous antibodies.

Herein, "monoclonal antibody" refers to an antibody obtained from a group of substantially homogeneous antibodies, that is, an antibody group wherein the antibodies constituting the group are homogeneous except for naturally occurring mutants that exist in a small amount. Monoclonal antibodies are highly specific and interact with a single antigenic site. Furthermore, each monoclonal antibody targets a single antigenic determinant (epitope) on an antigen, as compared to common polyclonal antibody preparations that typically contain various antibodies against diverse antigenic determinants. In addition to their specificity, monoclonal antibodies are advantageous in that they are produced from hybridoma cultures not contaminated with other immunoglobulins. The qualifier "monoclonal" indicates a characteristic of antibodies obtained from a substantially homogeneous group of antibodies, and does not specify antibodies produced by a particular method. For example, a monoclonal antibody to be used in the present invention can be produced by, for example, hybridoma methods (Kohler and Milstein, Nature 256:495, 1975) or recombination methods (U.S. Patent No. 4,816,567). The monoclonal antibodies used in the present invention can be also isolated from a phage antibody library (Clackson et al., Nature 352:624-628, 1991; Marks et al., J. Mol. Biol. 222:581-597, 1991). The monoclonal antibodies of the present invention particularly comprise "chimeric" antibodies (immunoglobulins), wherein a part of a heavy (H) chain and/or light (L) chain is derived from a specific species or a specific antibody class or subclass, and the remaining portion of the chain is derived from another species, or another antibody class or subclass. Furthermore, mutant antibodies and antibody fragments thereof are also comprised in the present invention (U.S. Patent No. 4,816,567; Morrison et al., Proc. Natl. Acad. Sci. USA 81:6851-6855, 1984).

Herein, "mutant antibody" refers to an antibody comprising a variant amino acid sequence in which one or more amino acid residues have been altered. For example, the variable region of an antibody can be modified to improve its biological properties, such as antigen binding. Such modifications can be achieved by site-directed mutagenesis (see Kunkel, Proc. Natl. Acad. Sci. USA 82: 488 (1985)), PCR-based mutagenesis, cassette mutagenesis, and the like. Such mutants comprise an amino acid sequence which is at least 70% identical to the amino acid sequence of a heavy or light chain variable region of the antibody, more preferably at least 75%, even more preferably at least 80%, still more preferably at least 85%, yet more preferably at least 90%, and most preferably at least 95% identical. Herein, sequence identity is defined as the percentage of residues identical to those in the antibody's original amino acid sequence, determined after the sequences are aligned and gaps are appropriately introduced to maximize the sequence identity as necessary.

Specifically, the identity of one nucleotide sequence or amino acid sequence to another can be determined using the algorithm BLAST, by Karlin and Altschul (Proc. Natl. Acad. Sci. USA, 90: 5873-5877, 1993). Programs such as BLASTN and BLASTX were developed based on this algorithm (Altschul et al., J. Mol. Biol. 215: 403-410, 1990). To analyze nucleotide sequences according to BLASTN based on BLAST, the parameters are set, for example, as score= 100 and wordlength= 12. On the other hand, parameters used for the analysis of amino acid sequences by BLASTX based on BLAST include, for example, score= 50 and wordlength= 3. Default parameters for each program are used when using the BLAST and Gapped BLAST programs. Specific techniques for such analyses are known in the art (see the website of the National Center for Biotechnology Information (NCBI), Basic Local Alignment Search Tool (BLAST); http://www.ncbi.nlm.nih.gov)

Polyclonal and monoclonal antibodies can be prepared by methods known to those skilled in the art. For example, the antibodies can be prepared by the methods described below.

For animal immunization, PCIs including the entire PCI amino acid sequence and partial peptides thereof prepared by recombinant DNA techniques or chemical synthesis can be used. The amino acid sequence of human PCI and those from other mammals are known (Suzuki, K. *et al.,* J. Biol. Chem. 1987, 262:611-616). Mammalian PCIs include but are not limited to, for example, mouse, rat, and bovine PCIs (Zechmeister-Machhart, M., *et al*., Gene, 186, (1), 61-66, 1997; Wakita, T., *et al*., FEBS Lett., 429, 263-268, (3), 1998; Yuasa, J., *et al*., Thromb. Haemost. 83, (2), 262-267, 2000). Recombinant PCI proteins can be prepared, for example, by the methods described in the Examples. As the antigen for immunization, PCI itself, or its partial peptides, can be used without modification, or after being conjugated with a carrier protein. When a carrier protein is used, for example, the antigen PCI is first coupled with the carrier protein (for example, thyroglobulin), and then an adjuvant is added thereto. Such adjuvants include Freund's complete and incomplete adjuvants and the like, any of which can be combined with the antigen.

An antigen prepared as described above is administered into a mammal, such as a mouse, rat, hamster, guinea pig, horse, monkey, rabbit, goat, and sheep. This immunization can be performed by any existing method, including typically used intravenous injections, subcutaneous injections, and intraperitoneal injections. There are no restrictions as to the immunization intervals. Immunization may be carried out at intervals of several days to several weeks, preferably four to 21 days. A mouse can be immunized, for example, at a single dose of 10 to 100 µg (for example, 20 to 60 µg) of the antigen protein.

Before the first immunization, and three to seven days after the second and subsequent immunizations, blood is collected from the animal, and the serum is analyzed for antibody titer. To promote an immune response, an aggregating agent such as alum is preferably used. In general, selected mammalian antibodies have sufficiently high antigen binding affinity. Antibody affinity can be determined using a saturation binding assay, an enzyme-linked immunosorbent assay (ELISA), or a competitive assay (for example, radioimmunoassay).

Polyclonal antibodies can be screened by a conventional crosslinking analysis, such as that described in "Antibodies, A Laboratory Manual (Cold Spring Harbor Laboratories, Harlow and David Lane edit. (1988))". An alternative method is, for example, epitope mapping (Champe et al., J. Biol. Chem. 270:1388-1394 (1995)). A preferred method for determining polypeptide or antibody titers comprises quantifying antibody-binding affinity. In other embodiments, methods for assessing one or more biological properties of an antibody are also used in addition to or in place of the methods for determining antibody-binding affinity. Such analytical methods are particularly useful because they demonstrate the therapeutic effectiveness of antibodies. When an antibody exhibits an improved property in such analyses, its binding affinity is generally, but not always, also enhanced.

Hybridomas which are used to prepare monoclonal antibodies can be obtained, for example, by the method of Milstein et al. (Kohler, G., and Milstein, C., Methods Enzymol. 1981, 73, 3-46). Myeloma cells to be fused with antibody-producing cells may be cell lines derived from any of the various animals, such as mice, rats, and humans, which are generally available to those skilled in the art. The cell lines to be used are drug-resistant, and cannot survive in a selective medium (e.g., HAT medium) in an unfused state, but can survive in a fused state. 8-azaguanine-resistant cell lines are generally used, which are deficient in hypoxanthine-guanine-phosphoribosyl transferase and cannot grow in a hypoxanthine-aminopterin-thymidine (HAT) medium. Preferred myeloma cells include a variety of known cell lines, for example, P3x63Ag8.653 (J. Immunol. (1979) 123: 1548-1550), P3x63Ag8U.1 (Current Topics in Microbiology and Immunology (1978) 81: 1-7), NS-1 (Kohler, G. and Milstein, C., Eur. J. Immunol. (1976) 6: 511-519), MPC-11 (Margulies, D. H. et al., Cell (1976) 8: 405-415), SP2/0 (Shulman, M. et al., Nature (1978) 276: 269-270), F0 (de St. Groth, S. F. et al., J. Immunol. Methods (1980) 35: 1-21), S194 (Trowbridge, I. S., J. Exp. Med. (1978) 148: 313-323), R210 (Galfre, G. et al., Nature (1979) 277: 131-133), and P3U1 (J. Exp. Med. 1979, 150:580; Curr Top Microbiol. Immunol. 1978, 81:1). Human myeloma and mouse-human heteromycloma cell lines can also be used to produce human monoclonal antibodies (Kozbar, J. Immunol. 133:3001 (1984); Brodeur et al., Monoclonal Antibody Production Techniques and Application, pp.51-63 (Marcel Dekker, Inc., New York, 1987)). Antibody-producing cells are collected, for example, from animals sacrificed two to three days after the final immunization. Antibody-producing cells include spleen cells, lymph node cells, and peripheral blood cells. Spleen cells are generally used. Specifically, tissues such as spleens or lymph nodes are excised or collected from the various animals described above. Then, the tissues are crushed and the resulting material is suspended in a medium or buffer, such as PBS, DMEM, or RPMI1640, followed by filtration with a stainless mesh or the like. This is then centrifuged to obtain antibody-producing cells of interest.

The above-described myeloma cells and antibody-producing cells are then fused. Cell fusion is achieved by contacting the myeloma cells with the antibody-producing cells at a ratio of 1:1 to 1:20 in a medium for animal cell culture, such as MEM, DMEM, and RPMI-1640, at 30 to 37°C for 1-15 minutes in the presence of a fusion-promoting agent. To promote cell fusion, the antibody-producing cells and the myeloma cells may be fused using a commercially available cell-fusion device, using a fusion-promoting agent, such as polyethylene glycol (mean molecular weight 1,000 to 6,000 (Da)) or polyvinyl alcohol, or a virus for fusion, such as Sendai virus.

Hybridomas of interest are selected from the cells after cell fusion. The selection methods include methods using selective propagation of cells in a selective medium. Specifically, a cell suspension is diluted with an appropriate medium, and then the cells are plated on to microtiter plates. An aliquot of selection medium (for example, HAT medium) is added to each well, and then the cells are cultured while the selection medium is appropriately exchanged. The cells grown as a result can be saved as hybridomas.

In another embodiment, antibodies or antibody fragments can be isolated from an antibody phage library, produced by using the technique reported by McCafferty et al. (Nature 348:552-554 (1990)). Clackson et al. (Nature 352:624-628 (1991)) and Marks et al. (J. Mol. Biol. 222:581-597 (1991)) reported on the respective isolation of mouse and human antibodies from phage libraries. There are also reports that describe the production of high affinity (nM range) human antibodies based on chain shuffling (Marks et al., Bio/Technology 10:779-783 (1992)), and combinatorial infection and in vivo recombination, which are methods for constructing large-scale phage libraries (Waterhouse et al., Nucleic Acids Res. 21:2265-2266 (1993)). These technologies can also be used to isolate monoclonal antibodies, in place of the conventional hybridoma technology for monoclonal antibody production.

Preferably, the neutralizing anti-PCI antibodies of the present invention can be selected by the screening method described below:

### 1st screening

To select antibodies which bind to PCI, each antibody is assessed for its binding specificity using a known technique, such as EIA (enzyme immunoassay), RIA (radioimmunoassay), ELISA (enzyme-linked immunosorbent assay), HTRF (homogenous time-resolved fluorescence), or fluorescence immunoassay (Antibodies A Laboratory Manual. Ed Harlow, David Lane, Cold Spring Harbor Laboratory, 1988).

### 2nd screening

An aPC/PCI assay and/or a Thr/TM/PCI assay are carried out to select antibodies that exhibit relatively strong inhibition against PCI. The aPC/PCI assay is for assaying PCI's inhibitory effect on the above-described aPC activity, and the Thr/TM/PCI assay is for assaying PCI's inhibitory effect on the aPC production (PC activation) by Thr/TM complex as described above. The degree of PCI activity inhibited is measured to select antibodies exhibiting relatively high degrees of inhibition. For example, when the assay comprises the use of antibodies prepared from antibody-producing cells (for example, hybridomas), the antibody-producing cells which produce antibodies comprising the activity of interest are identified and cloned by the limiting dilution method. The clones are grown using standard methods (Goding, Monoclonal Antibodies: Principals an Practice, pp.59-103, Academic Press, 1986). The cells may be cultured in a medium, for example, D-MEM or RPIM-1640 medium. Such hybridomas can be cloned by repeating the screening, which comprises selecting hybridomas that produce stronger anti-PCI neutralizing antibodies. The present invention relates to hybridomas producing antibodies of the present invention.

In the above aPC/PCI assay using hybridoma culture supernatants (1/5 (vol/vol)), the value is set at 100 in the absence of PCI, and at 0 when a hybridoma culture medium (for example, HAT medium) is used in place of the hybridoma culture supernatant. Antibody-producing hybridomas which have a relative value of PCI-inhibiting activity of preferably 45 or higher, more preferably 48 or higher, even more preferably 50 or higher, still more preferably 60 or higher, still more preferably 70 or higher, still more preferably 80 or higher, and yet still more preferably 90 or higher, are selected. The present invention provides hybridomas with a relative value of PCI-inhibiting activity of preferably 45 or higher, more preferably 48 or higher, more preferably 50 or higher, more preferably 60 or higher, more preferably 70 or higher, more preferably 80 or higher, and more preferably 90 or higher, in the aPC/PCI assay using culture supernatants (aPC/PCI: 1/5 (vol/vol)) . The aPC/PCI hybridoma culture supernatant assay is carried out using, for example, the methods described in the Examples.

Antibodies can be purified from hybridoma culture supernatants according to conventional methods. In the aPC/PCI assay where the value is set at 100% in the absence of antibody and at 0% in the absence of PCI, in order to achieve 50% inhibition, the antibodies of the present invention have a concentration of preferably 100 µg/ml or lower, more preferably 80 µg/ml or lower, even more preferably 60 µg/ml or lower, still more preferably 50 µg/ml or lower, still more preferably 40 µg/ml or lower, still more preferably 25 µg/ml or lower, still more preferably 15 µg/ml or lower, and yet still more preferably 12.5 µg/ml or lower. Alternatively, at an antibody concentration of 25 µg/ml, the antibodies of the present invention have a relative PCI inhibition value of preferably 40% or higher, more preferably 50% or higher, even more preferably 60% or higher, still more preferably 70% or higher, and yet still more preferably 80% or higher in the same aPC/PCI assay. The aPC/PCI assay of purified antibodies can be carried out, for example, by the methods described in the Examples. The 50% inhibition concentration can be determined by performing the assay at various antibody concentrations, plotting a graph, and determining the antibody concentration which corresponds to 50% inhibition from the graph.

Furthermore, the antibodies of the present invention preferably have Thr/TM/PCI-inhibiting activity. In the Thr/TM/PCI assay where the value is set at 100% in the absence of antibody and at 0% in the absence of PCI, in order to achieve 50% inhibition, the antibodies of the present invention have a concentration of preferably 200 µg/ml or lower, more preferably 150 µg/ml or lower, even more preferably 100 µg/ml or lower, still more preferably 80 µg/ml or lower, still more preferably 50 µg/ml or lower, still more preferably 30 µg/ml or lower, and yet still more preferably 25 µg/ml or lower. Alternatively, at an antibody concentration of 25 µg/ml, the antibodies of the present invention have a relative PCI inhibition value of preferably 10% or higher, more preferably 20% or higher, even more preferably 30% or higher, still more preferably 40% or higher, and yet still more preferably 50% or higher in the same Thr/TM/PCI assay. The Thr/TM/PCI assay of purified antibodies can be carried out, for example, by the methods described in the Examples. The 50% inhibition concentration can be determined by carrying out the assay at various antibody concentrations, plotting a graph, and determining the antibody concentration which corresponds to 50% inhibition from the graph.

The antibodies of the present invention can be any antibody that has PCI-inhibiting activity determined by either aPC/PCI assay or Thr/TM/PCI assay, and preferably have PCI-inhibiting activities determined by both of the aPC/PCI assay and the Thr/TM/PCI assay. Specifically, the antibodies of the present invention include antibodies whose respective 50% inhibition concentrations in the aPC/PCI assay and the Thr/TM/PCI assay described above are preferably 100 and 200 µg/ml or lower, more preferably 80 and 150 µg/ml or lower, even more preferably 60 and 100 µg/ml or lower, still more preferably 50 and 80 µg/ml or lower, still more preferably 40 and 50 µg/ml or lower, still more preferably 25 and 30 µg/ml or lower, still more preferably 15 and 25 µg/ml or lower, and yet still more preferably 12.5 and 25 µg/ml.

In general, antibodies exhibiting stronger PCI binding are considered as the more preferable antibodies of the present invention. The dissociation constant (KD) for the interaction between PCI and an antibody of the present invention is preferably 50 nM or less, more preferably 20 nM or less, even more preferably 10 nM or less, still more preferably 5 nM or less, still more preferably 3 nM or less, still more preferably 1 nM or less, still more preferably 0.8 nM or less, still more preferably 0.6 nM or less, still more preferably 0.4 nM or less, and yet still more preferably 0.2 nM or less. Kinetic parameters for the binding, such as dissociation constant, binding rate constant (ka), dissociation rate constant (kd), and maximal binding (Rmax), can be determined, for example, by a surface plasmon resonance analysis such as BIACORE.

Furthermore, the antibodies of the present invention preferably have activity to suppress the blood-mediated inactivation of aPC. The antibodies of the present invention suppress preferably 10% or more, more preferably 15% or more, even more preferably 20% or more, still more preferably 25% or more, and yet still more preferably 30% or more of the aPC inactivation by blood. Such suppression level is defined as the suppression rate of inactivation (%), and is expressed as a relative value between 0% (aPC activity when inactivated by blood) and 100% (aPC activity without inactivation).

The inactivation suppression rate may be determined under optimal conditions by appropriately changing the antibody concentration. Specifically, the rate can be determined as follows: 10 µL of 10 µg/mL aPC (for example, SIGMA, #P-2200) solution is combined with 40 µL of an antibody solution (e.g., a hybridoma culture supernatant, yielded during hybridoma screening) or a control solution without antibody (e.g., culture supernatant of myeloma cells, or HAT medium). The resulting mixture is incubated at room temperature for a certain period of time (for example, for 60 minutes). 50 µL of blood plasma (e.g., standard human plasma) is added to the mixture and also incubated at room temperature for a certain period of time (for example, for 60 minutes). 50 µL of APTT reagent (e.g., DADE BEHRING, GAA-200A) is added to the mixture. The blood coagulation time for an aPC sample incubated without blood plasma is determined by adding aPC to blood plasma immediately prior to the addition of APTT reagent. For example, 50 µL of 20 mmol/L CaCl2 (e.g., DADE BEHRING, GMZ-310) is added to the solution after incubation at 37°C for three minutes, and the time required for coagulation is then determined. Blood coagulation time can be determined using an automatic analyzer for blood coagulation (e.g., Amelung, KC-10A), or such.

Coagulation time (a) is taken as 100% when aPC incubated without blood plasma is added, and coagulation time (b) is taken as 0% when aPC incubated with blood plasma is added after incubation with a control solution without antibody (e.g., the supernatant of myeloma cell culture or HAT medium, as described above). Based on the coagulation time (c), when aPC incubated with blood plasma is added after incubation with an antibody solution such as hybridoma culture supernatant, the antibody solution such as the hybridoma culture supernatant, is assessed for its activity to extend coagulation time: (inactivation suppression rate (%) = {(c-b)/(a-b)} × 100). As this value increases, the activity of suppressing aPC inactivation is judged to be higher. Likewise, when aPC activity is assessed using a substrate compound, such as S-2366, the inactivation suppression rate (%) can be determined by assessing aPC activity in comparison with the activity of aPC inactivated by blood plasma but incubated without antibody, and the activity of aPC incubated without blood plasma.

The antibodies of the present invention can be, for example, any antibody that binds to a PCI portion involved in the interaction with or selectivity to aPC or thrombin. The PCI portion which interacts with thrombin and aPC is Arg³⁵⁴-Ser³⁵⁵ on human PCI after signal peptide is cleaved (Suzuki, K. et al., J. Biol. Chem. 1987,262: 611-616). The PCI portion associated with thrombin selectivity is Phe³⁵³, and the PCI portion associated with aPC selectivity is Thr³⁵² (Cooper, S.T. et al., Biochemistry 1995, 34: 12991-12997; Cooper, S.T. and Church, F.C., Biochemica et Biophysica Acta, 1246, 29-33, 1995). The antibodies of the present invention include, for example, antibodies binding to any one of these amino acids in PCI or amino acids in the vicinity thereof (for example, within 10 amino acids). Such an antibody can be prepared by synthesizing an oligopeptide encompassing a PCI portion of interest and using it as antigen to immunize animals for antibody production.

One of the PCI functions is to inhibit aPC activity, which depends on heparin, and PCI residues: Arg²⁷⁸, Arg³⁶², and Lys²⁷⁶ participate in the heparin-mediated PCI-aPC interaction (Lei Shen, *et al*., Thromb. Haemost., 82, 72-79, 1999). Thus, antibodies that bind to these amino acids or amino acids in the vicinity thereof become candidate antibodies for suppressing the PCI-mediated inhibition of aPC.

Methods for preparing monoclonal antibodies from the obtained hybridomas include standard cell culture methods and methods comprising ascites production. In cell culture methods, hybridomas are cultured for two to 14 days under standard culture conditions (for example, at 37°C at 5% CO₂ atmosphere), in a culture medium for animal cells, such as RPMI-1640 or MEM containing 10 to 20% fetal calf serum, or serum-free medium, and antibodies are then prepared from the culture supernatant. In the method comprising ascites production, hybridomas are administered to the peritoneal cavities of mammalian individuals of the same species as that from which the myeloma cells are derived, and the hybridomas proliferate into large quantities. Ascites or serum is then collected after one to four weeks. To enhance ascites production, for example, pristane (2,6,10,14-tetramethylpentadecane) may be pre-administered into the peritoneal cavity.

Antibodies to be used in the present invention can be purified by a method appropriately selected from known methods, such as the protein A-Sepharose method, protein G-Sepharose method, hydroxyapatite chromatography, salting-out method with sulfate, ion exchange chromatography, and affinity chromatography, or by the combined use of the same.

The present invention may use recombinant antibodies, produced by gene engineering. The genes encoding the antibodies obtained by a method described above are isolated from the hybridomas. The genes are inserted into an appropriate vector, and then introduced into a host (see, e.g., Carl, A. K. Borrebaeck, James, W. Larrick, Therapeutic Monoclonal Antibodies, Published in the United Kingdom by Macmillan Publishers Ltd, 1990). Specifically, using a reverse transcriptase, cDNAs encoding the variable regions (V regions) of the antibodies are synthesized from the mRNAs of hybridomas. After obtaining the DNAs encoding the variable regions of antibodies of interest, they are ligated with DNAs encoding desired constant regions (C regions) of the antibodies, and the resulting DNA constructs are inserted into expression vectors. Alternatively, the DNAs encoding the variable regions of the antibodies may be inserted into expression vectors comprising the DNAs of the antibody C regions. These are inserted into expression vectors so that the genes are expressed under the regulation of an expression regulatory region, for example, an enhancer and a promoter. Then, host cells are transformed with the expression vectors to express the antibodies. The present invention provides cells expressing antibodies of the present invention. The cells expressing antibodies of the present invention include cells and hybridomas transformed with a gene of such an antibody.

The particularly preferred antibodies of the present invention are antibodies that have antibody-binding sites fully or partially overlapped with an antibody comprising the variable region of any one of the monoclonal antibodies (PC19G8, PC23A7, PC23D8, PC30G1, PC31E2, PC31F1, and PC39C6) isolated in the Examples. Herein, such an antibody is referred to as an antibody that binds to practically the same site on PCI as one of the monoclonal antibodies (PC19G8, PC23A7, PC23D8, PC30G1, PC31E2, PC31F1, or PC39C6). Whether two antibodies bind to an identical site on an antigen protein can be determined by, for example, competition experiments. Specifically, when the binding between PCI and a first anti-PCI antibody is competitively inhibited by a second anti-PCI antibody, the first and the second antibodies are judged to bind to an identical site on the antigen. For example, an antibody which competes for the antigen binding site with any of the PC19G8, PC23A7, PC23D8, PC30G1, PC31E2, PC31F1, and PC39C6 antibodies, or an antibody comprising an H chain variable region comprising the amino acid sequence of SEQ ID NO: 8, 9, 10, 11, 12, 13, or 14 and a corresponding L chain variable region comprising the amino acid sequence of SEQ ID NO: 15, 16, 17, 18, 19, 20, or 21, is included in the antibodies of the present invention. Alternatively, an antibody that binds to practically the same site on PCI as one of the monoclonal antibodies described above can also be obtained, by using partial PCI peptides to analyze the epitope of a monoclonal antibody by known epitope mapping techniques, and using a peptide containing the identified epitope as an antigen to prepare a binding antibody. Such an antibody is expected to produce the same inhibitory effect against aPC production and/or aPC activity as the antibodies isolated in the Examples. Thus, the present invention also includes antibodies which bind to practically the same PCI site as the antibodies isolated in the Examples, wherein the isolated antibodies have the activity to inhibit the PCI inhibitory effect on aPC activity and/or aPC production by Thr/TM complex.

The antibodies of the present invention also include antibodies which comprise the complementarity-determining regions (CDRs) of any of the monoclonal antibodies isolated in the Examples (PC19G8, PC23A7, PC23D8, PC30G1, PC31E2, PC31F1, or PC39C6), or complementarity-determining regions functionally equivalent thereto. The term "functionally equivalent" refers to comprising amino acid sequences similar to the amino acid sequences of CDRs of any of the monoclonal antibodies isolated in the Examples, and having the activity of inhibiting PCI function by binding thereto. The term "CDR" refers to a region in an antibody variable region (also called "V region"), and determines the specificity of antigen binding. The H chain and L chain each have three CDRs, designated from the N terminus as CDR1, CDR2, and CDR3. There are four regions flanking these CDRs: these regions are referred to as "framework", and their amino acid sequences are highly conserved. The CDRs can be grafted into other antibodies, and thus a recombinant antibody can be prepared by combining CDRs with the framework of a desired antibody. One or more amino acids of a CDR can be modified without losing the ability to bind to its antigen. For example, one or more amino acids in a CDR can be substituted, deleted, and/or added.

An amino acid residue is preferably mutated into one that allows the properties of the amino acid side-chain to be conserved. Examples of the properties of amino acid side chains comprise: hydrophobic amino acids (A, I, L, M, F, P, W, Y, V), hydrophilic amino acids (R, D, N, C, E, Q, G, H, K, S, T), and amino acids comprising the following side chains: aliphatic side-chains (G, A, V, L, I, P); hydroxyl group-containing side-chains (S, T, Y); sulfur atom-containing side-chains (C, M); carboxylic acid- and amide-containing side-chains (D, N, E, Q); base-containing side-chains (R, K, H); and aromatic-containing side-chains (H, F, Y, W). (The letters within parenthesis indicate the one-letter amino acid codes.) Amino acid substitutions within each group are called conservative substitutions. It is well known that a polypeptide comprising a modified amino acid sequence in which one or more amino acid residues is deleted, added, and/or substituted can retain the original biological activity (Mark D.F. et al., Proc. Natl. Acad. Sci. U.S.A. 81: 5662-5666 (1984); Zoller M.J. and Smith M., Nucleic Acids Res. 10: 6487-6500 (1982); Wang A. et al., Science 224: 1431-1433; Dalbadie-McFarland G. et al., Proc. Natl. Acad. Sci. U.S.A. 79: 6409-6413 (1982)). The number of mutated amino acids is not limited, but in general, the number falls within 40% of amino acids of each CDR, and preferably within 35%, and still more preferably within 30% (e.g., within 25%). The identity of amino acid sequences can be determined as described herein.

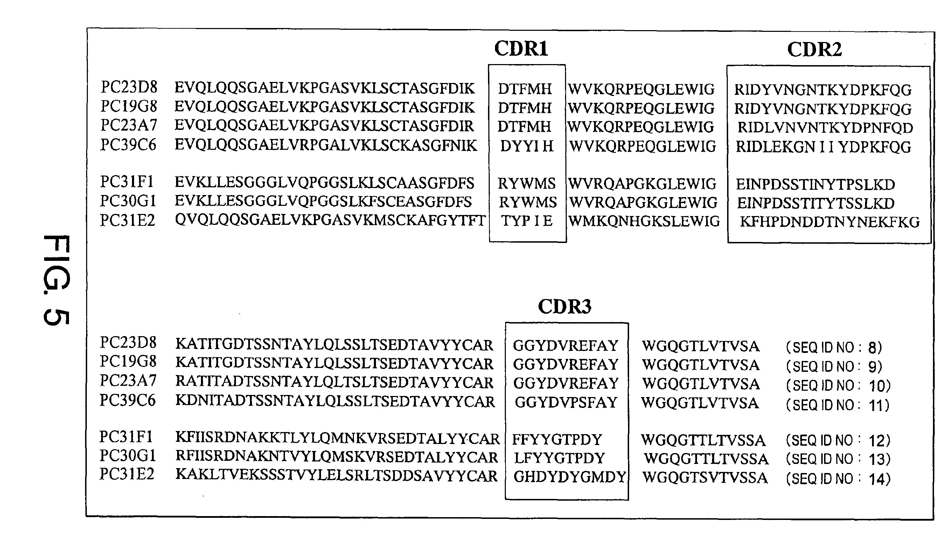

The antibodies of the present invention include, for example, an antibody that comprises three CDRs comprising amino acid sequences: D(T/Y)(F/Y)(M/I)H (SEQ ID NO: 49), RID(Y/L)(V/E)(N/K)(G/V)N(T/I)(K/I)YDP(K/N)FQ(G/D) (SEQ ID NO: 50), and GGYDV(R/P)(E/S)FAY (SEQ ID NO: 51) (wherein the slash dividing the amino acids implies that either of the amino acids may be present), or their functionally equivalent CDRs. The amino acid sequences indicated above correspond, respectively, to antibody H chain CDR1, CDR2, and CDR3. The PCI-neutralizing antibodies of the present invention can be prepared by inserting the CDRs into positions corresponding to CDR1, CDR2, and CDR3 in the framework of a desired H chain variable region. Specific examples of the preferred amino acid sequences of antibody H chain CDRs are: DTFMH (SEQ ID NO: 22) and DYYIH (SEQ ID NO: 23) for CDR1; RIDYVNGNTKYDPKFQG (SEQ ID NO: 26), RIDLVNVNTKYDPNFQD (SEQ ID NO: 27), and RIDLEKGNIIYDPKFQG (SEQ ID NO: 28) for CDR2; GGYDVREFAY (SEQ ID NO: 32) and GGYDVPSFAY (SEQ ID NO: 33) for CDR3. In addition, the amino acids of each CDR described above may be appropriately modified by substitution and the like. For example, the scope of the present invention encompasses conservative substitutions of the CDR amino acids. More specifically, the CDRs may comprise combinations of CDR1, 2, and 3 from the H chains of monoclonal antibodies PC23D8, PC19G8, PC23A7, and PC39C6 (see Fig. 5). Such an antibody is expected to have PCI-neutralizing activity equivalent to that of the clones described above.

The L chain variable region of an antibody may be appropriately combined with an antibody comprising an H chain CDR described above. The preferred L chain CDRs include combinations of CDRs comprising the amino acid sequences of SA(T/S)SS(LN)(I/S)YMH (SEQ ID NO: 55), STSNLASGVPA (SEQ ID NO: 56), and RSSYPFT (SEQ ID NO: 57), and functionally equivalent CDRs thereof. The amino acid sequences correspond, respectively, to CDR1, CDR2, and CDR3 of an antibody L chain. The L chain CDRs may also be used independently of the H chain described above. These CDRs are inserted into positions corresponding to CDR1, CDR2, and CDR3 in the framework of a desired L chain variable region. Specific examples of the preferred amino acid sequences of antibody L chain CDRs include, but are not limited to: SATSSLIYMH (SEQ ID NO: 37) and SASSSVSYMH (SEQ ID NO: 38) for CDR1, STSNLASGVPA (SEQ ID NO: 42) for CDR2, and RSSYPFT (SEQ ID NO: 46) for CDR3.

Specifically, the antibodies of the present invention includes those comprising the following H chain complementarity determining regions, and which have the activity to inhibit PCI's inhibitory effect on aPC activity and/or aPC production by the Thr/TM complex.
(a) complementarity determining regions consisting of the amino acid sequences of SEQ ID NOs: 49, 50, and 51.
(b) complementarity determining regions consisting of amino acid sequences with conservative substitutions of arbitrary amino acids in SEQ ID NOs: 49, 50, and 51.
(c) complementarity determining regions consisting of amino acid sequences with substitutions, deletions, and/or additions of three amino acids or less in SEQ ID NO: 49, eight amino acids or less in SEQ ID NO: 50, and five amino acids or less in SEQ ID NO: 51.
(d) complementarity determining regions consisting of amino acid sequences having 70% or higher identity to each of SEQ ID NOs: 49, 50, and 51.

The number of modified amino acids in the sequence of SEQ ID NO: 49 in (c) is preferably two or less, and more preferably one. The number of modified amino acids in the sequence of SEQ ID NO: 50 in (c) is preferably seven or less, more preferably six or less, even more preferably five or less, still more preferably four or less, yet still more preferably three or less. The number of modified amino acids in the sequence of SEQ ID NO: 51 in (c) is preferably four or less, more preferably three or less, even more preferably two or less, still more preferably one. The identity in (d) is preferably 75% or higher, more preferably 80% or higher, even more preferably 90% or higher, still more preferably 95% or higher.

Furthermore, the antibodies of the present invention also include those comprising the following L chain complementarity determining regions, and which have the activity to inhibit PCI's inhibitory effect on aPC activity and/or aPC production by the Thr/TM complex.
(a) The complementarity determining region comprising the amino acid sequences of SEQ ID NOs: 55, 56, and 57.
(b) The complementarity determining region comprising amino acid sequences with conservative substitutions of arbitrary amino acids in SEQ ID NOs: 55, 56, and 57.
(c) The complementarity determining region comprising amino acid sequences with substitutions, deletions, and/or additions of five amino acids or less in SEQ ID NO: 55, five amino acids or less in SEQ ID NO: 56, and four amino acids or less in SEQ ID NO: 57.
(d) The complementarity determining region comprising amino acid sequences having 70% or higher identity to each of SEQ ID NOs: 55, 56, and 57.

The number of modified amino acids in the sequence of SEQ ID NO: 55 in (c) is preferably four or less, more preferably three or less, even more preferably two or less, and still more preferably one. The number of modified amino acids in the sequence of SEQ ID NO: 56 in (c) is preferably four or less, more preferably three or less, even more preferably two or less, still more preferably one. The number of modified amino acids in the sequence of SEQ ID NO: 57 in (c) is preferably three or less, more preferably two or less, still more preferably one. The amino acid identity in (d) is preferably 75% or higher, more preferably 80% or higher, even more preferably 90% or higher, still more preferably 95% or higher. The preferred antibodies of the present invention include, in particular, antibodies comprising both the H chain and the L chain complementarity determining regions described above.

The antibodies of the present invention also include an antibody that comprises CDRs comprising the amino acid sequences of RYWMS (SEQ ID NO: 52), EINPDSSTI(N/T)YT(P/S)SLKD (SEQ ID NO: 53), and (F/L)FYYGTPDY (SEQ ID NO: 54), or their functionally equivalent CDRs. As described above, the amino acid sequences indicated above correspond to CDR1, CDR2, and CDR3 of an antibody H chain, respectively. Specific examples of the preferred amino acid sequences of antibody H chain CDRs are: RYWMS (SEQ ID NO: 24) for CDR1, EINPDSSTINYTPSLKD (SEQ ID NO: 29) and EINPDSSTITYTSSLKD (SEQ ID NO: 30) for CDR2, and FFYYGTPDY (SEQ ID NO: 34) and LFYYGTPDY (SEQ ID NO: 35) for CDR3. Specifically, combinations of CDR1, 2, and 3 from the H chain of monoclonal antibody PC30G1 or PC31F1 can be used. Such antibodies are expected to have PCI-neutralizing activity equivalent to that of PC30G1 or PC31F1. In this case, it is preferable to combine them with L chain CDRs, such as CDRs comprising the amino acid sequences of KASQDVI(V/K)AVA (SEQ ID NO: 58), S(A/T)SYRYTGVPD (SEQ ID NO: 59), and HYSSPPWT (SEQ ID NO: 60), or functionally equivalent CDRs thereof. These amino acid sequences correspond to CDR1, CDR2, and CDR3 of an antibody L chain, respectively. The L chain CDRs may also be used independently of the H chain described above. Specific examples of the preferred amino acid sequences of L chain CDR include but are not limited to, KASQDVIVAVA (SEQ ID NO: 39) and KASQDVIKAVA (SEQ ID NO: 40) for CDR1, SASYRYTGVPD (SEQ ID NO: 43) and STSYRYTGVPD (SEQ ID NO: 44) for CDR2, and HYSSPPWT (SEQ ID NO: 47) for CDR3.

Specifically, the antibodies of the present invention include those comprising the following H chain complementarity determining regions, and which have the activity to inhibit PCI's inhibitory effect on aPC activity and/or aPC production by the Thr/TM complex.
(a) The complementarity determining region comprising the amino acid sequences of SEQ ID NOs: 52, 53, and 54.
(b) The complementarity determining region comprising amino acid sequences with conservative substitutions of arbitrary amino acids in SEQ ID NOs: 52, 53, and 54.
(c) The complementarity determining region comprising amino acid sequences with substitutions, deletions, and/or addition of three amino acids or less in SEQ ID NO: 52, and eight amino acids or less in SEQ ID NO: 53, and five or less amino acids of SEQ ID NO: 54.
(d) The complementarity determining region comprising amino acid sequences having 70% or higher identity to each of SEQ ID NOs: 52, 53, and 54.

The number of modified amino acids in the sequence of SEQ ID NO: 52 in (c) is preferably two or less, and more preferably one. The number of modified amino acids in the sequence of SEQ ID NO: 53 in (c) is preferably seven or less, more preferably six or less, even more preferably five or less, still more preferably four or less, yet still more preferably three or less. The number of modified amino acids in the sequence of SEQ ID NO: 54 in (c) is preferably four or less, more preferably three or less, even more preferably two or less, still more preferably one. The amino acid identity in (d) is preferably 75% or higher, more preferably 80% or higher, even more preferably 90% or higher, still more preferably 95% or higher.

The antibodies of the present invention also include those comprising the following L chain complementarity determining regions, and which have the activity to inhibit PCI's inhibitory effect on aPC activity and/or aPC production by the Thr/TM complex.
(a) The complementarity determining region comprising the amino acid sequences of SEQ ID NOs: 58, 59, and 60.
(b) The complementarity determining region comprising amino acid sequences with conservative substitutions of arbitrary amino acids in SEQ ID NOs: 58, 59, and 60.
(c) The complementarity determining region comprising amino acid sequences with substitutions, deletions, and/or additions of five amino acids or less in SEQ ID NO: 58, five amino acids or less in SEQ ID NO: 59, and four or less amino acids in SEQ ID NO: 60.
(d) The complementarity determining region comprising amino acid sequences having 70% or higher identity to each of SEQ ID NOs: 58, 59, and 60.

The number of modified amino acids in the sequence of SEQ ID NO: 58 in (c) is preferably four or less, more preferably three or less, even more preferably two or less, still more preferably one. The number of modified amino acids in the sequence of SEQ ID NO: 59 in (c) is preferably four or less, more preferably three or less, even more preferably two or less, still more preferably one. The number of modified amino acids in the sequence of SEQ ID NO: 60 in (c) is preferably three or less, more preferably two or less, still more preferably one. The amino acid identity in (d) is preferably 75% or higher, more preferably 80% or higher, even more preferably 90% or higher, still more preferably 95% or higher. Antibodies comprising both the H chain and the L chain complementarity determining regions described above are particularly preferred as the antibodies of the present invention.

The antibodies of the present invention also include antibodies which comprise CDRs comprising the amino acid sequences of TYPIE (SEQ ID NO: 25), KFHPDNDDTNYNEKFKG (SEQ ID NO: 31), and GHDYDYGMDY (SEQ ID NO: 36), or functionally equivalent CDRs thereof. As described above, these amino acid sequences correspond to CDR1, CDR2, and CDR3 of an antibody H chain, respectively. Such an antibody is expected to have PCI-neutralizing activity equivalent to that of PC31E2. In this case, it is preferred to combine them with L chain CDRs, for example, CDRs comprising the amino acid sequences of KASQSVDYDGDSYLN (SEQ ID NO: 41), GASNLESGTPA (SEQ ID NO: 45), and SNEDPPT (SEQ ID NO: 48), or functionally equivalent CDRs thereof. These amino acid sequences correspond to CDR1, CDR2, and CDR3 of an antibody L chain, respectively.

Specifically, the antibodies of the present invention include those comprising the following H chain complementarity determining regions, and which have the activity to inhibit PCI's inhibitory effect on aPC activity and/or aPC production by the Thr/TM complex.
(a) The complementarity determining region comprising the amino acid sequences of SEQ ID NOs: 25, 31, and 36.
(b) The complementarity determining region comprising amino acid sequences with conservative substitutions of arbitrary amino acids in SEQ ID NOs: 25, 31, and 36.
(c) The complementarity determining region comprising amino acid sequences with substitutions, deletions, and/or additions of three amino acids or less in SEQ ID NO: 25, eight amino acids or less in SEQ ID NO: 31, and five or less amino acids in SEQ ID NO: 36.
(d) The complementarity determining region comprising amino acid sequences having 70% or higher identity to each of SEQ ID NOs: 25, 31, and 36.

The number of modified amino acids in the sequence of SEQ ID NO: 25 in (c) is preferably two or less, and more preferably one. The number of modified amino acids in the sequence of SEQ ID NO: 31 in (c) is preferably seven or less, more preferably six or less, even more preferably five or less, still more preferably four or less, yet still more preferably three or less. The number of modified amino acids in the sequence of SEQ ID NO: 36 in (c) is preferably four or less, more preferably three or less, even more preferably two or less, still more preferably one. The amino acid identity in (d) is preferably 75% or higher, more preferably 80% or higher, even more preferably 90% or higher, still more preferably 95% or higher.

The antibodies of the present invention also include those comprising the following L chain complementarity determining regions, and which have the activity to inhibit PCI's inhibitory effect on aPC activity and/or aPC production by the Thr/TM complex.
(a) The complementarity determining region comprising the amino acid sequences SEQ ID NOs: 41, 45, and 48.
(b) The complementarity determining region comprising amino acid sequences with conservative substitutions of arbitrary amino acids in SEQ ID NOs: 41, 45, and 48.
(c) The complementarity determining region comprising amino acid sequences with substitutions, deletions, and/or additions of five amino acids or less in SEQ ID NO: 41, five amino acids or less in SEQ ID NO: 45, four amino acids or less in SEQ ID NO: 48.
(d) The complementarity determining region comprising amino acid sequences having 70% or higher identity to each of SEQ ID NOs: 41, 45, and 48.

The number of modified amino acids in the sequence of SEQ ID NO: 41 in (c) is preferably four or less, more preferably three or less, even more preferably two or less, still more preferably one. The number of modified amino acids in the sequence of SEQ ID NO: 45 in (c) is preferably four or less, more preferably three or less, even more preferably two or less, still more preferably one. The number of modified amino acids in the sequence of SEQ ID NO: 48 in (c) is preferably three or less, more preferably two or less, still more preferably one. The degree of identity in (d) is preferably 75% or higher, more preferably 80% or higher, even more preferably 90% or higher, still more preferably 95% or higher. Antibodies comprising both the H chain and the L chain complementarity determining regions described above are particularly preferred as the antibodies of the present invention.

The CDR amino acid sequences can be modified, for example, by synthesizing oligonucleotides encoding the amino acid sequence of a modified variable region comprising CDR, and preparing nucleic acids encoding the variable region by PCR using the oligonucleotides. Antibodies which comprise desired CDRs can be prepared by inserting the nucleic acid into an appropriate expression vector and expressing it. For example, the oligonucleotides are synthesized using mixed nucleotides to prepare a DNA library that encodes a variety of antibodies comprising CDRs with various amino acids introduced at certain positions. An antibody of the present invention can be isolated by selecting from the library a clone encoding an antibody which binds to PCI and suppresses its activity. The present invention relates to the nucleic acids encoding the antibodies of the present invention, vectors comprising these nucleic acids, and host cells comprising the nucleic acids or the vectors. The nucleic acids may be DNAs or RNAs. The vectors include known desired vectors, such as plasmids, phages, and viral vectors. The host cells include bacteria, yeasts, insects, plant cells, and mammalian cells.

In the present invention, recombinant antibodies artificially modified to reduce heterologous antigenicity against humans can be used. Examples include chimeric antibodies and humanized antibodies. These modified antibodies can be produced using known methods. A chimeric antibody includes an antibody comprising variable and constant regions of species that are different to each other, for example, an antibody comprising the antibody heavy chain and light chain variable regions of a nonhuman mammal such as a mouse, and the antibody heavy chain and light chain constant regions of a human. Such an antibody can be obtained by (1) ligating a DNA encoding a variable region of a mouse antibody to a DNA encoding a constant region of a human antibody; (2) incorporating this into an expression vector; and (3) introducing the vector into a host for production of the antibody.

A humanized antibody, which is also called a reshaped human antibody, is obtained by substituting an H or L chain complementarity determining region (CDR) of an antibody of a nonhuman mammal such as a mouse, with the CDR of a human antibody. Conventional genetic recombination techniques for the preparation of such antibodies are known (see, for example, Jones et al., Nature 321: 522-525 (1986); Reichmann et al., Nature 332: 323-329 (1988); Presta Curr. Op. Struct. Biol. 2: 593-596 (1992)). Specifically, a DNA sequence designed to ligate a CDR of a mouse antibody with the framework regions (FRs) of a human antibody is synthesized by PCR, using several oligonucleotides constructed to comprise overlapping portions at their ends. A humanized antibody can be obtained by (1) ligating the resulting DNA to a DNA that encodes a human antibody constant region; (2) incorporating this into an expression vector; and (3) transfecting the vector into a host to produce the antibody (see, European Patent Application No. EP 239,400, and International Patent Application No. WO 96/02576). Human antibody FRs that are ligated via the CDR are selected where the CDR forms a favorable antigen-binding site. The humanized antibody may comprise additional amino acid residue(s) that are not included in the CDRs introduced into the recipient antibody, nor in the framework sequences. Such amino acid residues are usually introduced to more accurately optimize the antibody's ability to recognize and bind to an antigen. For example, as necessary, amino acids in the framework region of an antibody variable region may be substituted such that the CDR of a reshaped human antibody forms an appropriate antigen-binding site (Sato, K. et al., Cancer Res. (1993) 53, 851-856).

Methods for obtaining human antibodies are also known. For example, desired human antibodies with antigen-binding activity can be obtained by (1) sensitizing human lymphocytes with antigens of interest or cells expressing antigens of interest in vitro; and (2) fusing the sensitized lymphocytes with human myeloma cells such as U266 (see Examined Published Japanese Patent Application No. (JP-B) Hei 1-59878). Alternatively, the desired human antibody can also be obtained by using an antigen to immunize a transgenic (Tg) animal that comprises a partial or entire repertoire of human antibody genes (see Nature Genetics 7:13-21 (1994); Nature Genetics 15:146-156 (1997); Nature 368:856-859 (1994); International Patent Application WO 93/12227, WO 92/03918, WO 94/02602, WO 94/25585, WO 96/34096, and WO 96/33735). Specifically, such Tg animals are created as follows: a nonhuman mammal in which the loci of heavy and light chains of an endogenous immunoglobulin have been disrupted, and instead, the loci of heavy and light chains of a human immunoglobulin have been introduced via Yeast artificial chromosome (YAC) vectors and the like, is obtained by creating knockout animals or Tg animals, or mating such animals. The immunoglobulin heavy chain loci can be functionally inactivated, for example, by introducing a defect at a certain site in a J region or C region (e.g., Cm region). The immunoglobulin light chains (e.g., k chain) can be functionally inactivated, for example, by introducing a defect at a certain site in a J region or C region, or a region comprising the J and C regions.

Such a humanized antibody can also be obtained from culture supernatants, by using genetic engineering technology to transform eukaryotic cells with cDNAs that encode each of the heavy and light chains of the antibody, or preferably vectors comprising these cDNAs, and then culturing the transformed cells that produce the recombinant human monoclonal antibody. The hosts are, for example, desired eukaryotic cells, preferably mammalian cells, such as CHO cells, lymphocytes, and myelomas.

Furthermore, techniques to obtain human antibodies by panning with a human antibody library are known. For example, the variable region of a human antibody is expressed as a single chain antibody (scFv) on the surface of a phage, using phage display method, and phages that bind to the antigen can be selected. By analyzing the genes of selected phages, the DNA sequences encoding the variable regions of human antibodies that bind to the antigen can be determined. If the DNA sequences of scFvs that bind to the antigen are identified, appropriate expression vectors comprising these sequences can be constructed, and then introduced into appropriate hosts and expressed to obtain human antibodies. Such methods are already well known (see WO 92/01047, WO 92/20791, WO 93/06213, WO 93/11236, WO 93/19172, WO 95/01438, and WO 95/15388).

When the antibody genes have been isolated and introduced into an appropriate host, hosts and expression vectors can be used in appropriate combination to produce the antibodies. As eukaryotic host cells, animal cells, plant cells, and fungal cells may be used. The animal cells include: (1) mammalian cells such as CHO, COS, myeloma, baby hamster kidney (BHK), HeLa, and Vero cells; (2) amphibian cells such as Xenopus oocytes; or (3) insect cells such as sf9, sf21, and Tn5, or silkworms. Known plant cells include cells derived from the Nicotiana genus such as Nicotiana tabacum, which can be callus cultured. Known fungal cells include yeasts such as the Saccharomyces genus, for example Saccharomyces cerevisiae, and filamentous fungi such as the Aspergillus genus, for example Aspergillus niger. Prokaryotic cells can also be used in production systems that utilize bacterial cells. Known bacterial cells include E. coli and Bacillus subtilis. The antibodies can be obtained by transferring the antibody genes of interest into these cells using transformation, and then culturing the transformed cells in vitro.

The isotypes of the antibodies of the present invention are not limited. The isotypes include, for example, IgG (IgG1, IgG2, IgG3, and IgG4), IgM, IgA (IgA1 and IgA2), IgD, and IgE, with IgG and IgM being the preferable ones. The antibodies of the present invention may also be antibody fragments comprising a portion responsible for antigen binding, or a modified fragment thereof. The term "antibody fragment" refers to a portion of a full-length antibody, and generally to a fragment comprising an antigen-binding site or a variable region. Such antibody fragments include, for example, Fab, F(ab')2, Fv, single-chain Fv (scFv) which comprises a heavy chain Fv and a light chain Fv coupled together with an appropriate linker, diabody (diabodies), linear antibodies, and multispecific antibodies prepared from antibody fragments. Previously, antibody fragments were produced by digesting natural antibodies with a protease; currently, methods for expressing them as recombinant antibodies using genetic engineering techniques are also known (see Morimoto et al., Journal of Biochemical and Biophysical Methods 24:107-117 (1992); Brennan et al., Science 229:81 (1985); Co, M. S. et al., J. Immunol., 1994, 152, 2968-2976; Better, M. & Horwitz, A. H., Methods in Enzymology, 1989, 178, 476-496, Academic Press, Inc.; Plueckthun, A. & Skerra, A., Methods in Enzymology, 1989, 178, 476-496, Academic Press, Inc.; Lamoyi, E., Methods in Enzymology, 1989, 121, 663-669; Bird, R. E. et al., TIBTECH, 1991, 9,132-137).

An "Fv" fragment is the smallest antibody fragment, and contains a complete antigen recognition site and a binding site. This region is a dimer (VH-VL dimer) wherein the variable regions of each of the heavy chain and light chain are strongly connected by a noncovalent bond. The three CDRs of each of the variable regions interact with each other to form an antigen-binding site on the surface of the VH-VL dimer. In other words, a total of six CDRs from the heavy and light chains function together as an antibody's antigen-binding site. However, a variable region (or a half Fv, which contains only three antigen-specific CDRs) alone is also known to be able to recognize and bind to an antigen, although its affinity is lower than the affinity of the entire binding site. Thus, a preferred antibody fragment of the present invention is an Fv fragment, but is not limited thereto. Such an antibody fragment may be a polypeptide which comprises an antibody fragment of heavy or light chain CDRs which are conserved, and which can recognize and bind its antigen.

A Fab fragment (also referred to as F(ab)) also contains a light chain constant region and heavy chain constant region (CH1). For example, papain digestion of an antibody produces two kinds of fragments: an antigen-binding fragment called the Fab fragment, which contain heavy chain and light chain variable regions forming a single antigen-binding site; and the remaining portion, which is called an "Fc" because it is readily crystallized. A Fab' fragment is different from a Fab fragment in that a Fab' fragment also has several residues derived from the carboxyl terminus of a heavy chain CH1 region, which contains one or more cysteine residues of the hinge region of an antibody. A Fab' fragment is, however, structurally equivalent to Fab in that both are antigen-binding fragments comprising the variable regions of a heavy chain and light chain, which form a single antigen-binding site. Herein, an antigen-binding fragment which comprises the variable regions of a heavy chain and light chain forming a single antigen-binding site, and which is equivalent to that obtained by papain digestion, is referred to as a "Fab-like antibody", even when it is not identical to the antibody fragment produced by protease digestion. Fab'-SH is Fab' with one or more cysteine residues having free thiol groups in its constant region. A F(ab') fragment is produced by cleaving the disulfide bond between the cysteine residues in the hinge region of F(ab')2. Other chemically crosslinked antibody fragments are also known to those skilled in the art. Pepsin digestion of an antibody yields two fragments; one is a F(ab')2 fragment which comprises two antigen-binding domains and can cross-react with antigens, and the other is the remaining fragment (referred to as pFc'). Herein, an antibody fragment equivalent to that obtained by pepsin digestion is referred to as a "F(ab')2-like antibody" when it comprises two antigen-binding sites and can crossreact with antigens. Such antibody fragments can also be produced, for example, by genetic engineering. Such antibody fragments can also be isolated, for example, from the antibody phage library described above. Alternatively, F(ab')2-SH fragments can be recovered directly from hosts, such as E. coli, and then allowed to form F(ab')2 fragments by chemical crosslinking (Carter et al., Bio/Technology 10:163-167 (1992)). In an alternative method, F(ab')2 fragments can be isolated directly from a culture of recombinant hosts.

Furthermore, antibodies for use in the present invention may be multispecific antibodies. A multispecific antibody is an antibody that has specificity to at least two different kinds of antigens. Although such a molecule usually binds to two antigens (i.e., a bispecific antibody), the "multispecific antibody" herein encompasses antibodies with specificity to more than two antigens (e.g., three antigens). The multispecific antibody can be a full-length antibody or fragment thereof (e.g., F(ab')2 bispecific antibody). A bispecific antibody can be prepared by crosslinking the heavy and light chains of two types of antibodies (HL pairs), or from bispecific-antibody-producing cells produced by fusing hybridomas that produce different monoclonal antibodies (Millstein et al., Nature 305:537-539 (1983)). Alternatively, a bispecific antibody can be prepared by genetic engineering. Specifically, the variable domain of an antibody with binding specificity is fused to the constant domain sequence of an immunoglobulin. The above-mentioned constant domain sequence preferably comprises at least a part of the hinge, CH2, and the CH3 regions of the heavy chain constant domain of the immunoglobulin. Preferably, the CH1 region of the heavy chain required for binding with the light chain is also included. A DNA encoding the immunoglobulin heavy chain fusion is inserted into an expression vector to transform an appropriate host organism. As necessary, a DNA encoding the immunoglobulin light chain is also inserted into an expression vector, different to that of the immunoglobulin heavy chain fusion, to transform the host organism. There are cases where the antibody yield increases when the chain ratio is not identical. In such cases, it is more convenient to insert each of the genes into separate vectors, since the expression ratio of each of the chains can be controlled. However, genes encoding a number of chains can also be inserted into one vector.

The term "diabody (Db)" refers to a bivalent antibody fragment constructed by gene fusion (for example, P. Holliger et al., Proc. Natl. Acad. Sci. USA 90: 6444-6448 (1993), EP 404,097, WO 93/11161). In general, a diabody is a dimer of two polypeptide chains. In each of the polypeptide chains, a light chain variable region (VL) and a heavy chain variable region (VH) in an identical chain are connected via a short linker, for example, a linker of about five residues, so that they cannot bind to each other. Because the linker between the two is short, the VL and VH in the same polypeptide chain cannot form a single chain V region fragment, but instead form a dimer. Thus, a diabody has two antigen-binding domains. When the VL and VH regions against two types of antigens (a and b) are combined to form VLa-VHb and VLb-VHa via a linker of about five residues, and then co-expressed, they are secreted as bispecific Dbs. The antibodies of the present invention may be such Dbs.

A single-chain antibody (also referred to as "scFv") can be prepared by linking a heavy chain V region and a light chain V region of an antibody (for a review of scFv, see Pluckthun "The Pharmacology of Monoclonal Antibodies" Vol. 113, eds. Rosenburg and Moore, Springer Verlag, New York, pp.269-315 (1994)). Methods for preparing single-chain antibodies are known in the art (see, for example, US Patent Nos. 4,946,778, 5,260,203, 5,091,513, and 5,455,030). In such scFvs, the heavy chain V region and the light chain V region are linked together via a linker, preferably, a polypeptide linker (Huston, J. S. et al., Proc. Natl. Acad. Sci. U.S.A, 1988, 85, 5879-5883). The heavy chain V region and the light chain V region in a scFv may be derived from the same antibody, or from different antibodies. The peptide linker used to ligate the V regions may be any single-chain peptide consisting of 12 to 19 residues. A DNA encoding a scFv can be amplified by PCR using, as a template, either the entire DNA, or a partial DNA encoding a desired amino acid sequence, selected from a DNA encoding the heavy chain of the above antibody or the V region thereof, and a DNA encoding the light chain of the above antibody or the V region thereof; and using a primer pair that defines the two ends. Further amplification can be subsequently conducted using a combination of the DNA encoding the peptide linker portion, and the primer pair that defines both ends of the DNA to be ligated to the heavy and light chain respectively. After constructing DNAs encoding scFvs, conventional methods can be used to obtain expression vectors comprising these DNAs, and hosts transformed by these expression vectors. Furthermore, scFvs can be obtained according to conventional methods using the resulting hosts. These antibody fragments can be produced in hosts by obtaining genes that encode the antibody fragments and expressing these as outlined above. Antibodies bound to various types of molecules, such as polyethylene glycols (PEGs), may be used as modified antibodies. Methods for modifying antibodies are already established in the art. The term "antibody" in the present invention also encompasses the above-described antibodies.

The antibodies obtained can be purified to homogeneity. The antibodies can be isolated and purified by a method routinely used to isolate and purify proteins. The antibodies can be isolated and purified by the combined use of one or more methods appropriately selected from column chromatography, filtration, ultrafiltration, salting out, dialysis, preparative polyacrylamide gel electrophoresis, and isoelectrofocusing, for example (Strategies for Protein Purification and Characterization: A Laboratory Course Manual, Daniel R. Marshak et al. eds., Cold Spring Harbor Laboratory Press (1996); Antibodies: A Laboratory Manual. Ed Harlow and David Lane, Cold Spring Harbor Laboratory, 1988). Such methods are not limited to those listed above. Chromatographic methods include affinity chromatography, ion exchange chromatography, hydrophobic chromatography, gel filtration, reverse-phase chromatography, and adsorption chromatography. These chromatographic methods can be practiced using liquid phase chromatography, such as HPLC and FPLC. Columns to be used in affinity chromatography include protein A columns and protein G columns. For example, protein A columns include Hyper D, POROS, and Sepharose F.F. (Pharmacia). Antibodies can also be purified by utilizing antigen binding, using carriers on which antigens have been immobilized.

The present invention provides PCI activity inhibitors which comprise the antibodies of the present invention. The present invention also relates to the use of the antibodies of the present invention to inhibit PCI activity. The present invention further relates to methods of inhibiting PCI activity which comprise the step of contacting PCI with an antibody of the present invention. PCI inhibition of aPC production and/or aPC activity can be suppressed by contacting PCI with an antibody of the present invention. The present invention also provides agents for enhancing the production and/or activity of aPC, which comprise the antibodies of the present invention. The present invention also relates to the use of the antibodies of the present invention for enhancing aPC production and/or activity. Through the step of contacting PCI with an antibody of the present invention, it is possible to suppress the decrease in endogenous or exogenous PC activation and/or aPC activity by inhibiting PCI activity. The antibodies of the present invention may be administered alone or in combination with PC and/or aPC.

aPC is known to comprise the activities of suppressing blood coagulation and inflammation. Thus, the effect of aPC in suppressing blood coagulation or inflammation can be enhanced by the step of administering a neutralizing anti-PCI antibody of the present invention. The present invention relates to methods for suppressing blood coagulation or inflammation, which comprise the step of administering an antibody of the present invention. The methods may additionally comprise the step of administering PC and/or aPC. In this case, it is possible to administer an antibody of the present invention which has been previously mixed with PC and/or aPC, or administer them separately. The therapeutic effect of aPC (e.g., the prevention and treatment of thrombosis and sepsis) can be enhanced by using a pharmaceutical formulation, which comprises an antibody of the present invention as an active ingredient. The phrase "comprises an antibody of the present invention as an active ingredient" means comprising an antibody of the present invention as at least one active ingredient, and does not indicate any limitation as to the content of the antibody of the present invention. The antibodies of the present invention are useful to prevent or treat diseases that have developed and/or advanced due to a decrease or deficiency of activated Protein C activity, and are particularly effective for preventing and/or treating diseases that have developed due to the enhancement of blood coagulation reaction and/or inflammatory reaction. Specific examples of such diseases include arterial thrombosis, venous thrombosis, disseminated intravascular coagulation (DIC) syndrome, and sepsis.

The present invention also provides kits comprising: (a) an antibody of the present invention, and (b) PC and/or aPC. Such kits can be used to prevent or treat diseases that have developed and/or advanced due to a decrease or deficiency of activated Protein C activity. In addition, the present invention provides kits for use in preventing or treating diseases that have developed and/or advanced due to a decrease or deficiency of activated Protein C activity, which comprise: (a) at least one item selected from the group consisting of PC, aPC, and an antibody of the present invention, and (b) a recording medium comprising a description of the use of the antibody in combination with PC and/or aPC in therapeutically effective amounts, or a link to such a description. Such diseases include diseases that have developed due to the enhancement of the blood coagulation reaction and/or inflammatory reaction, as described above, and specifically include arterial thrombosis, venous thrombosis, DIC, and sepsis. The kits are useful to increase the relative in vivo activity of endogenous or administered PC or aPC. Thus, the kits can be used to prevent and treat the above-described diseases. The recording medium may be a desirable recording medium, including printable media, such as paper and plastic, floppy disk (FD), compact disk (CD), digital video disk (DVD), and computer-readable recording media, such as a semiconductor memory. These media are typically instruction manuals attached to a kit, which may contain a description of the combined use of an antibody of the present invention and PC and/or aPC at therapeutically effective doses. A 'link' is defined as a connection with no direct description about the combined use of the antibody and PC and/or aPC at therapeutically effective doses, but that informs users of the location of the description via a label or such, allowing users to reach the description using the label. For example, an instruction manual that gives instructions or suggestions to refer to an attached sheet, URL, or the like, which contains the description. Such a link connects to the description through preferably three clicks or less (link depth 3 or less), more preferably two clicks (link depth 2), and more preferably one click (link depth 1).

The antibodies of the present invention can be administered either orally or parenterally, but are preferably administered parenterally. Specific examples include injections, nasal formulations, pulmonary formulations, and cutaneous formulations. For example, injections can be administered systemically or locally by intravenous injection, intramuscular injection, intraperitoneal injection, or subcutaneous injection. Furthermore, the method of administration can be appropriately selected according to the age and symptoms of the patient. A single dose can be selected, from within the range of 0.0001 mg to 1,000 mg per kg of body weight. Alternatively, the dose can be selected, from within the range of 0.001 to 100,000 mg/body for each patient. However, the dose of an antibody of the present invention is not limited to these examples.

The antibodies of the present invention can be formulated according to standard methods (see, for example, Remington's Pharmaceutical Science, latest edition, Mark Publishing Company, Easton, U.S.A), and may comprise pharmaceutically acceptable carriers and/or additives. Exemplary carriers include surfactants (for example, PEG and Tween), excipients, antioxidants (for example, ascorbic acid), coloring agents, flavoring agents, preservatives, stabilizers, buffering agents (for example, phosphoric acid, citric acid, and other organic acids), chelating agents (for example, EDTA), suspending agents, isotonizing agents, binders, disintegrators, lubricants, fluidity promoters, and corrigents. However, the carriers that may be employed in the present invention are not limited to this list. In fact, other commonly used carriers can be appropriately employed: light anhydrous silicic acid, lactose, crystalline cellulose, mannitol, starch, carmelose calcium, carmelose sodium, hydroxypropylcellulose, hydroxypropylmethylcellulose, polyvinylacetaldiethylaminoacetate, polyvinylpyrrolidone, gelatin, medium chain fatty acid triglyceride, polyoxyethylene hydrogenated castor oil 60, sucrose, carboxymethylcellulose, corn starch, inorganic salt, and so on. The composition may also comprise other low-molecular-weight polypeptides, proteins such as serum albumin, gelatin, and immunoglobulin, and amino acids such as glycine, glutamine, asparagine, arginine, and lysine. When the composition is prepared as an aqueous solution for injection, it can comprise an isotonic solution comprising, for example, physiological saline, dextrose, and other adjuvants, including, for example, D-sorbitol, D-mannose, D-mannitol, and sodium chloride, which can also contain an appropriate solubilizing agent, for example, alcohol (for example, ethanol), polyalcohol (for example, propylene glycol and PEG), and non-ionic detergent (polysorbate 80 and HCO-50).

If necessary, antibodies of the present invention may be encapsulated in microcapsules (microcapsules made of hydroxycellulose, gelatin, polymethylmethacrylate, and the like), and made into components of colloidal drug delivery systems (liposomes, albumin microspheres, microemulsions, nano-particles, and nano-capsules) (for example, see "Remington's Pharmaceutical Science 16th edition", Oslo Ed. (1980)). Moreover, methods for making sustained-release drugs are known, and these can be applied for the antibodies of the present invention (Langer et al., J. Biomed. Mater. Res. 15: 167-277 (1981); Langer, Chem. Tech. 12: 98-105 (1982); USP: 3,773,919; EP Patent Application No. 58,481; Sidman et al., Biopolymers 22: 547-556 (1983); EP: 133,988).

In addition, genes encoding the antibodies of the present invention may be used for gene therapy, by cloning into vectors for such use. Such vectors can be administered by direct injection using naked plasmids, and also by packaging in liposomes, producing as a variety of viral vectors such as retroviral vectors, adenovirus vectors, vaccinia virus vectors, poxvirus vectors, adenoassociated virus vectors, and HVJ vectors (Adolph, "Virus Genome Methods", CRC Press, Florida (1996)), or by coating onto carrier beads such as colloidal gold particles (for example, WO93/17706). However, any method can be used for administration, as long as the antibodies are expressed in vivo and exercise their function. Preferably, a sufficient dose may be administered by a suitable parenteral route (such as injecting intravenously, intraperitoneally, subcutaneously, percutaneously, or into adipose tissues or mammary glands, inhalation, intramuscular injection, infusion, gas-induced particle bombardment (using electron guns and such), or through mucosa, for example, using nose drops). Alternatively, genes encoding the antibodies of the present invention may be administered into cells ex vivo using liposome transfection, particle bombardment (USP: 4,945,050), or viral infection, and the cells may be reintroduced into animals.

### Brief Description of the Drawings

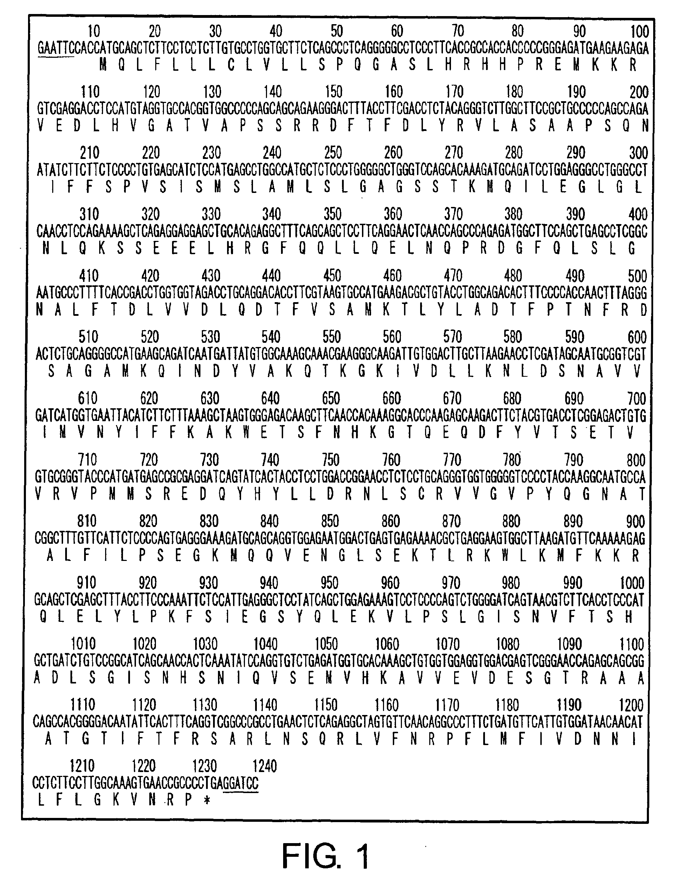

Fig. 1 shows the nucleotide sequence of PCI cDNA (without tag). The nucleotide sequence of a full-length PCI gene is shown in this figure. The EcoRI and BamHI recognition sequences (underlined) have been added, respectively, to the 5' and 3' ends of the sequence, for insertion in between the EcoRI and BamHI sites on the animal cell expression vector pCHOI. In addition, a Kozak sequence has been attached to the 5' end of the initiation codon to improve transcription effciency. The nucleotide sequence is assigned as SEQ ID NO: 4, and its amino acid sequence is assigned as SEQ ID NO: 5.

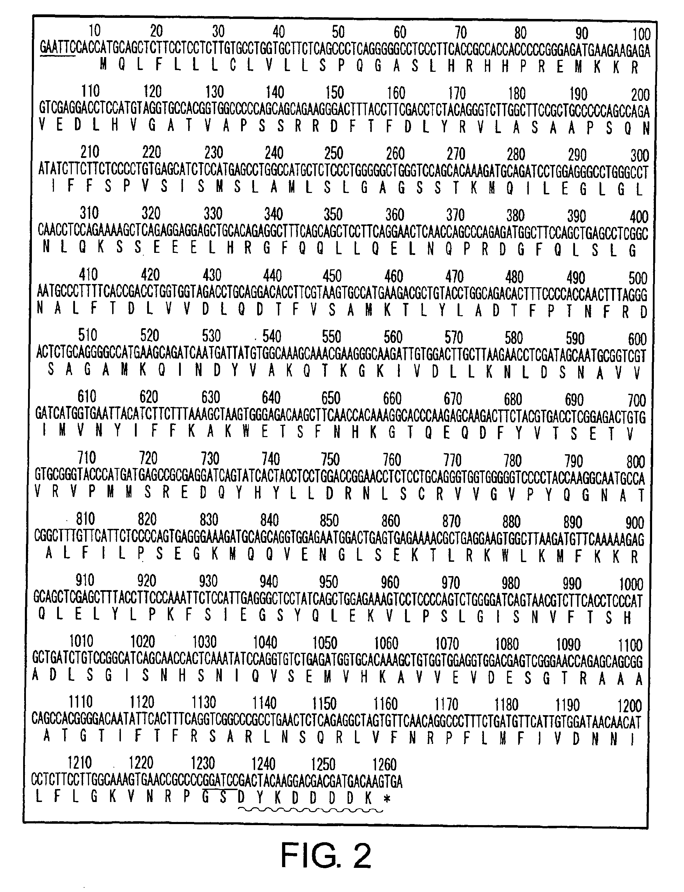

Fig. 2 shows the nucleotide sequence of PCI cDNA (with FLAG tag). The nucleotide sequence of a Flag-tagged PCI gene is shown in this figure. A Flag sequence (wavy line) has been attached to the 3' end of the full-length PCI gene by inserting the full-length PCI-encoding cDNA between the EcoRI and BamHI sites on the animal cell expression vector pCHO2-FLAG. The nucleotide sequence is assigned as SEQ ID NO: 6, and its amino acid sequence is assigned as SEQ ID NO: 7.

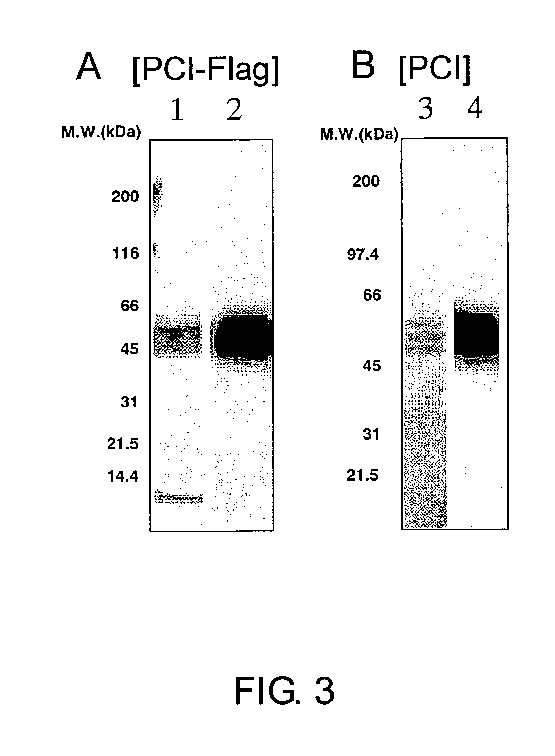

Fig. 3 shows photographs of PCI-Flag and PCI by SDS-PAGE and Western blot analyses. (A) PCI-Flag (lanes 1 and 2) and (B) non-tagged PCI (lanes 3 and 4) were fractionated by SDS-PAGE, and detected by Coomassie Blue staining (lanes 1 and 3) and by Western blotting using an anti-PCI antibody (lanes 2 and 4).

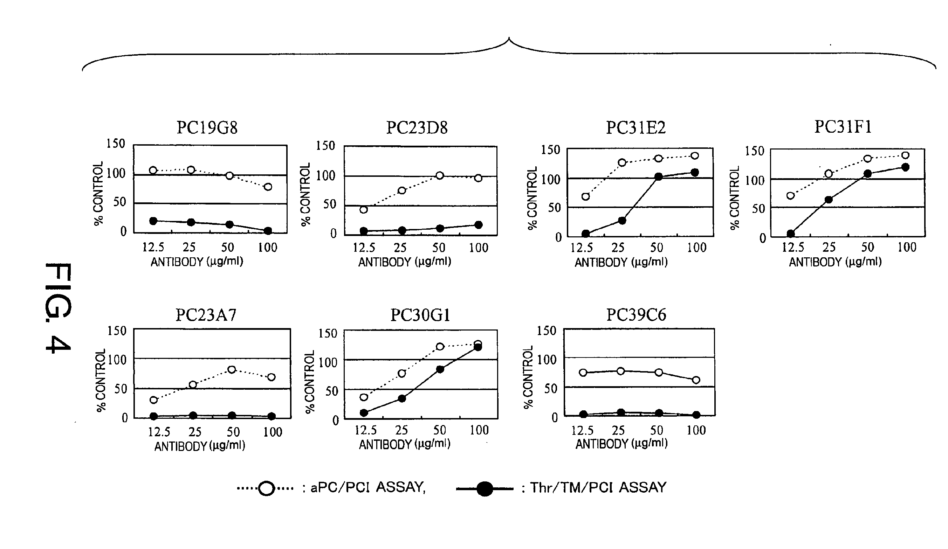

Fig. 4 shows a comparison of neutralizing activities of anti-PCI antibodies by aPC/PCI and Thr/TM/PCI assays. Open circle shows aPC/PCI assay results and closed circle shows Thr/TM/PCI assay results. The level of activity is expressed as a relative value, between 100% activity in the absence of PCI and 0% activity in the presence of PCI but in the absence of antibody.
Fig. 5 shows the amino acid sequences of the H chains of the respective anti-PCI neutralizing antibodies. CDR 1, 2, and 3 are boxed. The amino acid sequences in the figure correspond to, respectively, SEQ ID NOs: 8-14, from the top.

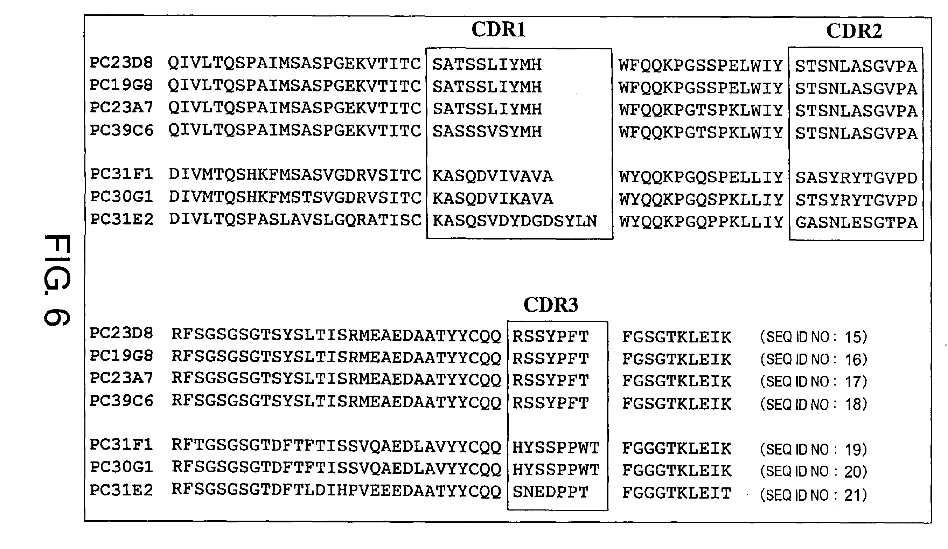

Fig. 6 shows the amino acid sequences of the L chains of the respective anti-PCI neutralizing antibodies. CDR 1, 2 and 3 are boxed. The amino acid sequences in the figure correspond to, respectively, SEQ ID NOs: 15-21, from the top.

### Best Mode for Carrying out the Invention

Herein below, the present invention will be specifically described using examples, however, it is not to be construed as being limited thereto. All publications cited herein are incorporated by reference in their entireties.

### [Example 1] Construction of PCI expression vectors

### 1.1 Cloning of PCI gene

A full-length PCI gene was cloned by PCR using the primers indicated below.

PCI-up: 5'- ACG AAT TCC ACC ATG CAG CTC TTC CTC (SEQ ID NO: 1)

PCI-low: 5'- CTG GAT CCT CAG GGG CGG TTC ACT TTG C (SEQ ID NO: 2)

The human PCI gene which comprises the entire ORF containing an EcoRI sequence at the 5' end and a BamHI sequence at the 3' end was amplified by PCR, using the primers described above and Human kidney marathon ready cDNA (Clontech) as a template. The amplified DNA fragment was digested with EcoRI and BamHI, and inserted between the cleaved EcoRI and BamHI sites in the animal cell expression vector pCHOI. The nucleotide sequence of the PCI gene in vector was determined to select a plasmid containing the desired sequence, thereby completing the construction of the pCHOI-PCI vector (Fig. 1).

A Flag-tagged PCI expression vector (PCI-Flag) was constructed as described below. The PCI gene was amplified by PCR using the pCHOI-PCI vector as a template, and PCI-up and PCI-low2 primers. PCI-low2: 5'- TTG GAT CCG GGG TTC ACT TTG CCA AG (SEQ ID NO: 3) The DNA fragment was digested with EcoRI and BamHI, and then inserted between the cleaved EcoRI and BamHI sites on the animal cell expression vector pCHOII-Flag, which comprises a Flag tag immediately after the cloning site. The nucleotide sequence was confirmed, thus completing the construction of pCHOII-PCI-Flag (Fig. 2).

### 1.2 Establishment of PCI and PCI-Flag producing cell lines

The pCHOI-PCI and pCHOII-PCI-Flag plasmids were linearized by PvuI digestion. 30 µg of the DNAs were introduced into CHO cells (DXB11 strain) by electroporation. Then, the cells were cultured in α(-)MEM (nucleic acid-free) (GIBCO BRL CAT# 12561-056) containing 5% FBS (GIBCO BRL CAT#10099-141). Cell lines producing PCI or PCI-Flag were selected. The selected cell lines were cultured in the same medium containing a final concentration of 50 nM MTX to establish cell lines that highly produce PCI and PCI-Flag. The expressions of PCI and PCI-Flag were confirmed using an anti-PCI antibody (Affinity Biologicals CAT#GAPCI-IG).

### [Example 2] Purification of PCI-Flag

The PCI-Flag-overexpressing CHO cell lines were cultured in roller bottles (1700 cm²) using α(-)MEM (nucleic acid-free) containing 5% FBS. The cells were cultured until confluent (37°C, 0.5 rpm), and then the media were removed. The cells were washed with PBS, and cultured in CHO-S-SFMII medium (GIBCO BRL CAT#12052-098) for 72 hours. The culture supernatant obtained was centrifuged to remove cell debris, filtered through 0.45-µm filters, and then used in the purification step described below. The culture supernatant was loaded onto a CM Sepharose Fast Flow column (Amersham CAT# 17-0719-01) equilibrated with 50 mM Tris buffer (pH 7.0) containing 0.05% Tween20. The column was washed, and then eluted with the same buffer containing 400 mM NaCl. The eluted fraction was diluted to adjust the NaCl concentration to 200 mM. The diluted fraction was loaded onto an anti-Flag M2 agarose affinity gel column (SIGMA CAT#A-2220) equilibrated with 50 mM Tris buffer (pH7.4) containing 150 mM NaCl and 0.05% Tween20. The column was eluted with 100 mM glycine buffer (pH3.5) containing 0.05% Tween20. The eluted fraction was immediately neutralized with 1 M Tris buffer (pH8.0). Then, the fraction containing PCI-Flag was loaded onto a CM Sepharose Fast Flow column equilibrated with 50 mM phosphate buffer (pH 7.0) containing 0.05% Tween20, and eluted with the same buffer containing 400 mM NaCl for the purpose of solvent displacement. The eluted sample was then concentrated by ultrafiltration using Centricon YM-3 (Amicon) to prepare PCI-Flag. The purified protein was fractionated by SDS-PAGE, and then confirmed by Coomassie Blue staining, and by Western analysis using an anti-PCI antibody after transferring onto a PVDF membrane (Fig. 3A).

### [Example 3] Purification of PCI

The PCI-overexpressing CHO cell line was cultured using roller bottles (1700 cm²) and the culture supernatant was prepared by the same method as described above. The culture supernatant was loaded onto a CM Sepharose Fast Flow column equilibrated with 50 mM Tris buffer (pH 7.0) containing 0.05% Tween20. After washing, the column was eluted with the same buffer containing 400 mM NaCl. Then, the PCI-containing fraction was loaded onto a HiTrap Heparin HP (Amersham CAT# 17-0407-01) column equilibrated with 10 mM phosphate buffer (pH 7.0) containing 0.05% Tween20. The sample was eluted with a NaCl step gradient (concentration from 0 mM to 1000 mM). The eluted fraction was loaded onto a Superdex 200 26/60 column (Amersham CAT# 17-1071-01) and fractionated by molecular weight. For the solvent, PBS containing 0.01% Tween 20 (PBS-T) was used. This process was repeated twice for PCI purification. The purified protein was fractionated by SDS-PAGE, and then confirmed by Coomassie Blue staining, and by Western analysis using an anti-PCI antibody after transferring onto a PVDF membrane (Fig. 3B).

### [Example 4] Preparation of anti-PCI antibodies having PCI-neutralizing activity

### 4.1 Immunization and preparation of hybridomas

Five Balb/c mice (female, 13-week old; Charles River Japan, Inc.) were immunized with PCI-Flag as an antigen according to conventional methods. 100 µg/head of the antigen was used for the first immunization. The antigen was emulsified using FCA [Freund's complete adjuvant (H37 Ra), Difco (3113-60), Becton Dickinson (cat#231131)], and injected subcutaneously into the mice. After two weeks, 50 µg/head of the antigen was emulsified using FIA [Freund's incomplete adjuvant, Difco(0639-60), Becton Dickinson (cat#263910)] and injected subcutaneously into the mice. Then, booster immunization was carried out five times in total at two-week intervals. The final immunization was carried out by injecting the PBS-diluted antigen (50 µg/head) in the caudal vein or subcutaneously. After the anti-PCI antibody titer was confirmed to be elevated in sera by ELISA, using immunoplates coated with 100 µl of 0.5 µg/ml PCI per well, the final immunization was carried out by injecting the No. 2 mouse in the caudal vein and the No. 4 mouse subcutaneously. Murine myeloma P3U1 cells and murine spleen cells were combined and fused using PEG1500 (Roche Diagonostic, cat# 783 641) according to conventional methods. The respective murine hybridoma cells were cultured in sixteen 96-well culture plates. HAT selection began on the following day, using HAT medium [10% FBS/RPMI 1640/1x HAT media supplement (SIGMA CAT# H-0262)/4% BM-Condimed H1 Hybridoma cloning supplement (Roche CAT# 1088947)]. 10 days after the fusion, culture supernatants were collected for ELISA screening. ELISA screening was carried out using immunoplates coated with 100 µl/well of 0.5 µg/ml PCI by the same method as described above for antibody titer assay.

### 4.2 Screening

### 4.2.1 ELISA

Positive wells were selected by ELISA screening using PCI. Then, the cells were cloned by expanding in 24-well plates and by limiting dilution (100 cells from a single positive well were placed into separate wells of a 96-well plate). The cloned hybridomas were expanded and antibodies were purified from the culture supernatants. 290 positive wells were selected and the cells were expanded in 24-well plates. Then, the first 111 wells exhibiting higher OD values in the primary screening were selected, and the cells were cloned by limiting dilution. From the 179 wells which had not been treated with limiting dilution, culture supernatants were collected and the cells were stored. Finally, from the 111 wells treated with limiting dilution, 81 clones stably producing antibodies were established.

### 4.2.2 aPC/PCI assay

In an aPC/PCI assay for determining the PCI-neutralizing activity, following the addition of a hybridoma culture supernatant or purified antibody and 5 µg/ml PCI, the reaction solution (50 mM Tris-HCl (pH8.0), 150 mM NaCl, 2 mM CaCl₂, 0.1% BSA, and 5 U/ml Heparin) was incubated at 37°C for 30 minutes. 0.25 µg/ml aPC was added to the mixture, which was then incubated at 37°C for another 30 minutes. 0.4 mM Spectrozyme aPC (American Diagnostica Inc.) was added to the mixture. After 2 hours of incubation at room temperature, the mixture was analyzed by colorimetry at 405 nm. (The concentrations indicated above are all final concentrations). 81 samples of monocloned hybridoma culture supernatants were analyzed by aPC/PCI assay, and clones in which aPC activity had been recovered by neutralizing the PCI activity were selected. From clones with strong PCI-neutralizing activity, 16 clones with the strongest PCI-neutralizing activity were selected, and then antibodies were purified from the culture supernatants using Protein G columns. aPC/PCI assays using the purified antibodies confirmed that eight of the 16 clones have a strong dose-dependent neutralizing activity. Fig. 4 shows dose-dependent curves for the seven clones among them.

### 4.2.3 Thrombin (Thr)/thrombomodulin (TM)/PCI assay

In a thrombin (Thr)/thrombomodulin (TM)/PCI assay for determining the PCI-neutralizing activity, a purified antibody, 5 µg/ml PCI, 2 nM Thr, and 10 nM TM were added to the reaction solution (50 mM Tris-HCl (pH 8.0), 150 mM NaCl, 2 mM CaCl₂, 0.1% BSA, and 5 U/ml heparin), which was then incubated at 37°C for 30 minutes, followed by addition of 0.73 µg/ml PC and incubation at 37°C for 50 minutes. Then, 0.875 µg/ml argatroban was added to the mixture to stop the reaction. 0.4 mM Spectrozyme aPC was added to the reaction solution, followed by 2 hours of incubation at room temperature, and colorimetry analysis at 405 nm. (The concentrations indicated above are all final concentrations).

The seven clones that had been confirmed to have neutralizing activity by the aPC/PCI assay were analyzed by the Thr/TM/PCI assay using purified antibodies. As a result, three (PC31E2, PC31F1, and PC30G1) out of the seven clones were confirmed to have a strong dose-dependent neutralizing activity (Fig. 4).

### 4.3 Purification of antibodies

Antibodies with an IgG1, IgG2a, or IgG2b isotype, were purified as follows. The hybridoma culture supernatants were loaded onto a Hi-Trap Protein G HP (Amersham CAT# 17-0404-01) equilibrated with 20 mM phosphate buffer (pH7.0). After washing, the columns were eluted with 0.1 M glycine buffer (pH2.7). The eluted fraction was immediately neutralized with 1 M Tris buffer (pH9.0). Fractions containing the antibody were pooled, and then dialyzed overnight against PBS containing 0.05% Tween20 for solvent displacement. Then, 0.02% NaN₃ was added to the dialyzed sample.

### 4.4 Isotyping of anti-PCI antibodies

Isotyping of anti-PCI antibodies was carried out using the ImmunoPure Monoclonal Antibody Isotyping Kit II (PIERCE CAT# 37502) according to the method described in the attached manual. Isotyping analysis of the established 81 anti-PCI antibody clones yielded 70 clones of IgG1, 6 clones of IgG2a, 4 clones of IgG2b, and 1 clone of IgM.

### 4.5 Kinetic analyses of anti-PCI antibodies using BIACORE

PCI-Flag was diluted to 25 µg/ml with 10 mM sodium acetate (pH 5.0), and then amine-coupled to a sensor chip CM5 (BIACORE; BR-1000-14) using an amine coupling kit (BIACORE; BR-1000-50) according to the method described in the kit. Approximately 3000 RU of PCI-Flag was immobilized onto the CM5 chip by this treatment. The kinetic analyses described below were performed on BIACORE 2000 using the sensor chip. Each anti-PCI antibody was diluted to 1.25, 2.5, 5, 10, and 20 µg/ml with HBS-EP buffer (BIACORE; BR-1001-88). After the chip was equilibrated with HBS-EP buffer, 40 µl of the antibody solution at each concentration was injected at a flow rate of 20 µl/min. In the association phase, the antibody was injected over 2 minutes. Then, instead of the antibody, HBS-EP buffer was injected over 2 minutes in the dissociation phase. After the dissociation phase, 40 µl of 10 mM HCl and then 40 µl of 0.05% SDS were injected continuously to regenerate the sensor chip. The sensorgrams obtained by the procedure described above were superimposed, and the binding rate constant (ka), dissociation rate constant (kd), dissociation constant (KD), and maximal binding (Rmax) were computed using a data analysis software (BIAevaluation, ver.3.0).

For the 8 clones found to exhibit strong PCI-neutralizing activity by aPC/PCI assay using the purified antibodies, BIACORE kinetic analyses were performed. The data revealed that many of the antibodies included exhibit relatively high affinities with a dissociation constant ranged from 10⁻⁹ to 10⁻¹⁰ M. Table 1 summarizes the characteristics of anti-PCI antibodies from the obtained clones.

**Table 1**

| Characteristics of neutralizing antibodies | | | | | | |
|---|---|---|---|---|---|---|
| Clone | Isotype | aPC/PCI | Thr/TM/PCI (+H) | Kinetic Parameter | | |
| | | | | ka (1/Ms) | kd (1/s) | KD (nM) |
| PC19G8 | IgG1 | + | - | 1.68E+05 | 2.13E-05 | 0.126 |
| PC23A7 | IgG2a | + | - | 1.51E+05 | 7.17E-05 | 0.473 |
| PC23D8 | IgG2a | + | - | 2.25E+05 | 6.59E-05 | 0.293 |
| PC30G1 | IgG1 | + | + | 1.82E+05 | 4.54E-05 | 0.249 |
| PC31E2 | IgG1 | + | + | 1.75E+05 | 3.13E-04 | 1.79 |
| PC31F1 | IgG1 | + | + | 1.53E+05 | 3.89E-05 | 0.254 |
| PC39C6 | IgG1 | + | - | 8.88E+04 | 4.89E-04 | 5.51 |

### [Example 5] Analysis of the H chains and the L chains of neutralizing anti-PCI antibodies

Using the RNeasy Plant Mini Kits (QIAGEN, Cat. No. 74904), total RNA was extracted from about 1x 10⁷ cells in each antibody-producing hybridoma. Then, cDNA was synthesized from the total RNA using the SMART RACE cDNA Amplification Kit (Clontech, Cat. No. K1811-1). The H chains and L chains were amplified by 5'-RACE PCR using the Advantage2 PCR Kit with primers specific to the IgG1 constant region for clones PC19G8, PC30G1, PC31E2, PC31F1, and PC39C6, or primers specific to the IgG2a constant region for clones PC23A7 and PC23D8. The amplified H chain and L chain DNA fragments were cloned into pGEM-T easy vector (Promega, Cat. No. A1360), and their nucleotide sequences were determined.

The obtained nucleotide sequences were analyzed, and amino acid sequences of the H chain and L chain variable regions are respectively shown in Figs. 5 and 6. The amino acid sequences of PC19G8 and PC23D8 were found to be identical, and therefore the antibodies were predicted to be derived from an identical clone. However, class switch was suspected to have taken place considering that the isotypes of PC19G8 and PC23D8 were IgG1 and IgG2a, respectively. PC23A7 and PC39C6 have sequences similar to the antibodies from the two clones described above. This suggests that the epitopes recognized by antibodies of the four clones are in close vicinity. On the other hand, the PC30G1 and PC31F1 sequences show low similarity to the four antibody clones described above. However, these two clones have similar sequences, and thus the epitopes for the two antibodies were predicted to be located in the vicinity of each other. The PC31E2 sequence was found to have low similarity to the other six clones. PC19G8, PC23A7, PC23D8, and PC39C6 suppressed only the aPC-PCI system, while PC30G1, PC31E2, and PC31F1 suppressed both the aPC-PCI and Thr-TM-PCI systems. Thus, presumably, the sequence-based categorization has closely reflected the pattern of PCI-neutralizing activity.

### Industrial Applicability

The present invention provides anti-PCI antibodies having PCI-neutralizing activity. The antibodies of the present invention have the activity to inhibit PCI's inhibitory effect on aPC production and enzymatic activity, and thus maintain aPC activity and its physiological functions, such as suppression of the activation of blood coagulation system and anti-inflammatory functions. The antibodies of the present invention can be used to prevent or treat diseases or disorders that have developed and/or advanced due to a decrease or deficiency of activated Protein C activity, in particular, thrombosis, sepsis, and the like, using aPC.

## Claims

1. An anti-PCI antibody, having at least any one of: (a) activity to inhibit an inhibitory effect of Protein C inhibitor (PCI) on activated Protein C (aPC) activity, or (b) activity to inhibit an inhibitory effect of Protein C inhibitor (PCI) on the production of activated Protein C (aPC) by thrombin /thrombomodulin (Thr/TM) complex.

2. An anti-PCI antibody, having both (a) activity to inhibit an inhibitory effect of Protein C inhibitor (PCI) on activated Protein C (aPC) activity, and (b) activity to inhibit an inhibitory effect of Protein C inhibitor (PCI) on the production of activated Protein C (aPC) by thrombin /thrombomodulin (Thr/TM) complex.

3. The antibody of claim 1 or 2, wherein the antibody competes for the antibody-binding site with an antibody comprising a variable region of an antibody selected from the group consisting of PC19G8, PC23A7, PC23D8, PC30G1, PC31E2, PC31F1, and PC39C6.

4. The antibody of claim 1 or 2, wherein the antibody comprises complementarity determining regions consisting of the amino acid sequences of any one of (a) to (f), or complementarity determining regions functionally equivalent thereto:
(a) the amino acid sequences of SEQ ID NOs: 49, 50, and 51;
(b) the amino acid sequences of SEQ ID NOs: 55, 56, and 57;
(c) the amino acid sequences of SEQ ID NOs: 52, 53, and 54;
(d) the amino acid sequences of SEQ ID NOs: 58, 59, and 60;
(e) the amino acid sequence of SEQ ID NOs: 25, 31, and 36; and
(f) the amino acid sequences of SEQ ID NOs: 41, 45, and 48.

5. The antibody of claim 1 or 2, wherein the antibody is selected from the group consisting of human antibodies, humanized antibodies, chimeric antibodies, antibody fragments, single-chain antibodies, and diabodies.

6. A composition comprising the antibody of claim 1 or 2 and a pharmaceutically acceptable carrier.

7. The composition of claim 6, further comprising Protein C and/or activated Protein C.

8. The composition of claim 6, wherein the composition is a pharmaceutical composition used to prevent or treat a disease that has developed and/or advanced due to a decrease or deficiency of activated Protein C activity.

9. The composition of claim 8, wherein the disease is caused by hypercoagulation and/or a hyperinflammatory reaction.

10. The composition of claim 9, wherein the disease caused by hypercoagulation and/or a hyperinflammatory reaction is selected from the group consisting of sepsis, disseminated intravascular coagulation syndrome, arterial thrombosis, and venous thrombosis.

11. A method for preventing or treating a disease that has developed and/or advanced due to a decrease or deficiency of activated Protein C activity, wherein the method comprises the step of administering (a) Protein C and/or activated Protein C, and (b) the antibody of claim 1 or 2.

12. A method for preventing or treating a disease that has developed and/or advanced due to a decrease or deficiency of activated Protein C activity, wherein the method comprises the step of administering the antibody of claim 1 or 2.

13. A kit used to prevent or treat a disease that has developed and/or advanced due to a decrease or deficiency of activated Protein C activity, wherein the kit comprises (a) the antibody of claim 1 or 2, and (b) Protein C, activated Protein C, or both.

14. A kit used to prevent or treat a disease that has developed and/or advanced due to a decrease or deficiency of activated Protein C activity, wherein the kit comprises (a) Protein C, activated Protein C, and the antibody of claim 1 or 2; (b) a recording medium comprising a description on the combined use of (i) a therapeutically effective amount of Protein C and/or activated Protein C and (ii) the antibody of claim 1 or 2, or a link to the description.


SEQUENCE LISTING

<110>  CHUGAI SEIYAKU KABUSHIKI KAISHA

<120>  Anti-PCI neutralizing antibodies

<130>  C1-A0226P

<150> JP   2003-011529
<151> 2003-01-20

<160>  60

<170>  PatentIn version 3.1

<210>  1
<211>  17
<212>  DNA
<213>  Artificial

<220>
<223>  Artificially synthesized sequence

<400>  1
acgaattcca ccatgcagct cttcctc                                          17

<210>  2
<211>  18
<212>  DNA
<213>  Artificial

<220>
<223>  Artificially synthesized sequence

<400>  2
ctggatcctc aggggcggtt cactttgc                                         18

<210> 3
<211> 16
<212> DNA
<213> Artificial

<220>
<223> Artificially synthesized sequence

<400> 3
ttggatccgg ggttcacttt gccaag 16

<210> 4
<211> 1237
<212> DNA
<213> Artificial

<220>
<223> Artificially synthesized sequence encoding human PCI

<220>
<221> CDS
<222> (11)..(1228)

<400> 4
gaattccacc atg cag ctc ttc ctc ctc ttg tgc ctg gtg ctt ctc agc 49
Met Gln Leu Phe Leu Leu Leu Cys Leu Val Leu Leu Ser
1               5                   10

cct cag ggg gcc tcc ctt cac cgc cac cac ccc cgg gag atg aag aag 97
Pro Gln Gly Ala Ser Leu His Arg His His Pro Arg Glu Met Lys Lys
    15                  20                  25

aga gtc gag gac ctc cat gta ggt gcc acg gtg gcc ccc agc agc aga 145
Arg Val Glu Asp Leu His Val Gly Ala Thr Val Ala Pro Ser Ser Arg
30                  35                  40                  45

agg gac ttt acc ttc gac ctc tac agg gtc ttg gct tcc gct gcc ccc 193
Arg Asp Phe Thr Phe Asp Leu Tyr Arg Val Leu Ala Ser Ala Ala Pro
                50                  55                  60

agc cag aat atc ttc ttc tcc cct gtg agc atc tcc atg agc ctg gcc 241
Ser Gln Asn Ile Phe Phe Ser Pro Val Ser Ile Ser Met Ser Leu Ala
            65                  70                  75

atg ctc tcc ctg ggg gct ggg tcc agc aca aag atg cag atc ctg gag 289
Met Leu Ser Leu Gly Ala Gly Ser Ser Thr Lys Met Gln Ile Leu Glu
        80                  85                  90

ggc ctg ggc ctc aac ctc cag aaa agc tca gag gag gag ctg cac aga 337
Gly Leu Gly Leu Asn Leu Gln Lys Ser Ser Glu Glu Glu Leu His Arg
    95                  100                 105

ggc ttt cag cag ctc ctt cag gaa ctc aac cag ccc aga gat ggc ttc 385
Gly Phe Gln Gln Leu Leu Gln Glu Leu Asn Gln Pro Arg Asp Gly Phe
110                 115                 120                 125

cag ctg agc ctc ggc aat gcc ctt ttc acc gac ctg gtg gta gac ctg 433
Gln Leu Ser Leu Gly Asn Ala Leu Phe Thr Asp Leu Val Val Asp Leu
                130                 135                 140

cag gac acc ttc gta agt gcc atg aag acg ctg tac ctg gca gac act 481
Gln Asp Thr Phe Val Ser Ala Met Lys Thr Leu Tyr Leu Ala Asp Thr
            145                 150                 155

ttc ccc acc aac ttt agg gac tct gca ggg gcc atg aag cag atc aat 529
Phe Pro Thr Asn Phe Arg Asp Ser Ala Gly Ala Met Lys Gln Ile Asn
        160                 165                 170

gat tat gtg gca aag caa acg aag ggc aag att gtg gac ttg ctt aag 577
Asp Tyr Val Ala Lys Gln Thr Lys Gly Lys Ile Val Asp Leu Leu Lys
    175                 180                 185

aac ctc gat agc aat gcg gtc gtg atc atg gtg aat tac atc ttc ttt 625
Asn Leu Asp Ser Asn Ala Val Val Ile Met Val Asn Tyr Ile Phe Phe
190                 195                 200                 205

aaa gct aag tgg gag aca agc ttc aac cac aaa ggc acc caa gag caa 673
Lys Ala Lys Trp Glu Thr Ser Phe Asn His Lys Gly Thr Gln Glu Gln
                210                 215                 220

gac ttc tac gtg acc tcg gag act gtg gtg cgg gta ccc atg atg agc 721
Asp Phe Tyr Val Thr Ser Glu Thr Val Val Arg Val Pro Met Met Ser
            225                 230                 235

cgc gag gat cag tat cac tac ctc ctg gac cgg aac ctc tcc tgc agg 769
Arg Glu Asp Gln Tyr His Tyr Leu Leu Asp Arg Asn Leu Ser Cys Arg
        240                 245                 250

gtg gtg ggg gtc ccc tac caa ggc aat gcc acg gct ttg ttc att ctc 817
Val Val Gly Val Pro Tyr Gln Gly Asn Ala Thr Ala Leu Phe Ile Leu
    255                 260                 265

ccc agt gag gga aag atg cag cag gtg gag aat gga ctg agt gag aaa 865
Pro Ser Glu Gly Lys Met Gln Gln Val Glu Asn Gly Leu Ser Glu Lys
270                 275                 280                 285

acg ctg agg aag tgg ctt aag atg ttc aaa aag agg cag ctc gag ctt 913
Thr Leu Arg Lys Trp Leu Lys Met Phe Lys Lys Arg Gln Leu Glu Leu
                290                 295                 300

tac ctt ccc aaa ttc tcc att gag ggc tcc tat cag ctg gag aaa gtc 961
Tyr Leu Pro Lys Phe Ser Ile Glu Gly Ser Tyr Gln Leu Glu Lys Val
            305                 310                 315

ctc ccc agt ctg ggg atc agt aac gtc ttc acc tcc cat gct gat ctg 1009
Leu Pro Ser Leu Gly Ile Ser Asn Val Phe Thr Ser His Ala Asp Leu
        320                 325                 330

tcc ggc atc agc aac cac tca aat atc cag gtg tct gag atg gtg cac    1057
Ser Gly Ile Ser Asn His Ser Asn Ile Gln Val Ser Glu Met Val His
    335                 340                 345

aaa gct gtg gtg gag gtg gac gag tcg gga acc aga gca gcg gca gcc    1105
Lys Ala Val Val Glu Val Asp Glu Ser Gly Thr Arg Ala Ala Ala Ala
350                 355                 360                 365

acg ggg aca ata ttc act ttc agg tcg gcc cgc ctg aac tct cag agg    1153
Thr Gly Thr Ile Phe Thr Phe Arg Ser Ala Arg Leu Asn Ser Gln Arg
                370                 375                 380

cta gtg ttc aac agg ccc ttt ctg atg ttc att gtg gat aac aac atc    1201
Leu Val Phe Asn Arg Pro Phe Leu Met Phe Ile Val Asp Asn Asn Ile
            385                 390                 395

ctc ttc ctt ggc aaa gtg aac cgc ccc tgaggatcc                      1237
Leu Phe Leu Gly Lys Val Asn Arg Pro
        400                 405

<210>  5
<211>  406
<212>  PRT
<213>  Artificial

<220>
<223>  Human PCI

<220>
<221>  sig_peptide
<222>  (1)..(19)

<400>  5
Met Gln Leu Phe Leu Leu Leu Cys Leu Val Leu Leu Ser Pro Gln Gly

1                   5                   10                  15

Ala Ser Leu His Arg His His Pro Arg Glu Met Lys Lys Arg Val Glu
                20                  25                  30

Asp Leu His Val Gly Ala Thr Val Ala Pro Ser Ser Arg Arg Asp Phe
            35                  40                  45

Thr Phe Asp Leu Tyr Arg Val Leu Ala Ser Ala Ala Pro Ser Gln Asn
        50                  55                  60

Ile Phe Phe Ser Pro Val Ser Ile Ser Met Ser Leu Ala Met Leu Ser
65                  70                  75                  80

Leu Gly Ala Gly Ser Ser Thr Lys Met Gln Ile Leu Glu Gly Leu Gly
                85                  90                  95

Leu Asn Leu Gln Lys Ser Ser Glu Glu Glu Leu His Arg Gly Phe Gln
            100                 105                 110

Gln Leu Leu Gln Glu Leu Asn Gln Pro Arg Asp Gly Phe Gln Leu Ser
        115                 120                 125

Leu Gly Asn Ala Leu Phe Thr Asp Leu Val Val Asp Leu Gln Asp Thr
    130                 135                 140

Phe Val Ser Ala Met Lys Thr Leu Tyr Leu Ala Asp Thr Phe Pro Thr
145                 150                 155                 160

Asn Phe Arg Asp Ser Ala Gly Ala Met Lys Gln Ile Asn Asp Tyr Val
                165                 170                 175

Ala Lys Gln Thr Lys Gly Lys Ile Val Asp Leu Leu Lys Asn Leu Asp
            180                 185                 190

Ser Asn Ala Val Val Ile Met Val Asn Tyr Ile Phe Phe Lys Ala Lys

        195                 200                 205

Trp Glu Thr Ser Phe Asn His Lys Gly Thr Gln Glu Gln Asp Phe Tyr
    210                 215                 220

Val Thr Ser Glu Thr Val Val Arg Val Pro Met Met Ser Arg Glu Asp
225                 230                 235                 240

Gln Tyr His Tyr Leu Leu Asp Arg Asn Leu Ser Cys Arg Val Val Gly
                245                 250                 255

Val Pro Tyr Gln Gly Asn Ala Thr Ala Leu Phe Ile Leu Pro Ser Glu
            260                 265                 270

Gly Lys Met Gln Gln Val Glu Asn Gly Leu Ser Glu Lys Thr Leu Arg
        275                 280                 285

Lys Trp Leu Lys Met Phe Lys Lys Arg Gln Leu Glu Leu Tyr Leu Pro
    290                 295                 300

Lys Phe Ser Ile Glu Gly Ser Tyr Gln Leu Glu Lys Val Leu Pro Ser
305                 310                 315                 320

Leu Gly Ile Ser Asn Val Phe Thr Ser His Ala Asp Leu Ser Gly Ile
                325                 330                 335

Ser Asn His Ser Asn Ile Gln Val Ser Glu Met Val His Lys Ala Val
            340                 345                 350

Val Glu Val Asp Glu Ser Gly Thr Arg Ala Ala Ala Ala Thr Gly Thr
        355                 360                 365

Ile Phe Thr Phe Arg Ser Ala Arg Leu Asn Ser Gln Arg Leu Val Phe
    370                 375                 380

Asn Arg Pro Phe Leu Met Phe Ile Val Asp Asn Asn Ile Leu Phe Leu

385                 390                 395                 400

Gly Lys Val Asn Arg Pro
                405

<210>  6
<211>  1261
<212>  DNA
<213>  Artificial

<220>
<223>  Artificially synthesized DNA encoding human PCI with Flag-tag

<220>
<221>  CDS
<222>  (11)..(1258)

<400>  6
gaattccacc atg cag ctc ttc ctc ctc ttg tgc ctg gtg ctt ctc agc      49
           Met Gln Leu Phe Leu Leu Leu Cys Leu Val Leu Leu Ser
           1               5                   10

cct cag ggg gcc tcc ctt cac cgc cac cac ccc cgg gag atg aag aag      97
Pro Gln Gly Ala Ser Leu His Arg His His Pro Arg Glu Met Lys Lys
    15                  20                  25

aga gtc gag gac ctc cat gta ggt gcc acg gtg gcc ccc agc agc aga      145
Arg Val Glu Asp Leu His Val Gly Ala Thr Val Ala Pro Ser Ser Arg
30                  35                  40                  45

agg gac ttt acc ttc gac ctc tac agg gtc ttg gct tcc gct gcc ccc      193
Arg Asp Phe Thr Phe Asp Leu Tyr Arg Val Leu Ala Ser Ala Ala Pro
                50                  55                  60

agc cag aat atc ttc ttc tcc cct gtg agc atc tcc atg agc ctg gcc      241

Ser Gln Asn Ile Phe Phe Ser Pro Val Ser Ile Ser Met Ser Leu Ala
            65                  70                  75

atg ctc tcc ctg ggg gct ggg tcc agc aca aag atg cag atc ctg gag 289
Met Leu Ser Leu Gly Ala Gly Ser Ser Thr Lys Met Gln Ile Leu Glu
        80                  85                  90

ggc ctg ggc ctc aac ctc cag aaa agc tca gag gag gag ctg cac aga 337
Gly Leu Gly Leu Asn Leu Gln Lys Ser Ser Glu Glu Glu Leu His Arg
    95                  100                 105

ggc ttt cag cag ctc ctt cag gaa ctc aac cag ccc aga gat ggc ttc 385
Gly Phe Gln Gln Leu Leu Gln Glu Leu Asn Gln Pro Arg Asp Gly Phe
110                 115                 120                 125

cag ctg agc ctc ggc aat gcc ctt ttc acc gac ctg gtg gta gac ctg 433
Gln Leu Ser Leu Gly Asn Ala Leu Phe Thr Asp Leu Val Val Asp Leu
                130                 135                 140

cag gac acc ttc gta agt gcc atg aag acg ctg tac ctg gca gac act 481
Gln Asp Thr Phe Val Ser Ala Met Lys Thr Leu Tyr Leu Ala Asp Thr
            145                 150                 155

ttc ccc acc aac ttt agg gac tct gca ggg gcc atg aag cag atc aat 529
Phe Pro Thr Asn Phe Arg Asp Ser Ala Gly Ala Met Lys Gln Ile Asn
        160                 165                 170

gat tat gtg gca aag caa acg aag ggc aag att gtg gac ttg ctt aag 577
Asp Tyr Val Ala Lys Gln Thr Lys Gly Lys Ile Val Asp Leu Leu Lys
    175                 180                 185

aac ctc gat agc aat gcg gtc gtg atc atg gtg aat tac atc ttc ttt 625
Asn Leu Asp Ser Asn Ala Val Val Ile Met Val Asn Tyr Ile Phe Phe
190                 195                 200                 205

aaa gct aag tgg gag aca agc ttc aac cac aaa ggc acc caa gag caa 673

Lys Ala Lys Trp Glu Thr Ser Phe Asn His Lys Gly Thr Gln Glu Gln
                210                 215                 220

gac ttc tac gtg acc tcg gag act gtg gtg cgg gta ccc atg atg agc 721
Asp Phe Tyr Val Thr Ser Glu Thr Val Val Arg Val Pro Met Met Ser
            225                 230                 235

cgc gag gat cag tat cac tac ctc ctg gac cgg aac ctc tcc tgc agg 769
Arg Glu Asp Gln Tyr His Tyr Leu Leu Asp Arg Asn Leu Ser Cys Arg
        240                 245                 250

gtg gtg ggg gtc ccc tac caa ggc aat gcc acg gct ttg ttc att ctc 817
Val Val Gly Val Pro Tyr Gln Gly Asn Ala Thr Ala Leu Phe Ile Leu
    255                 260                 265

ccc agt gag gga aag atg cag cag gtg gag aat gga ctg agt gag aaa 865
Pro Ser Glu Gly Lys Met Gln Gln Val Glu Asn Gly Leu Ser Glu Lys
270                 275                 280                 285

acg ctg agg aag tgg ctt aag atg ttc aaa aag agg cag ctc gag ctt 913
Thr Leu Arg Lys Trp Leu Lys Met Phe Lys Lys Arg Gln Leu Glu Leu
                290                 295                 300

tac ctt ccc aaa ttc tcc att gag ggc tcc tat cag ctg gag aaa gtc 961
Tyr Leu Pro Lys Phe Ser Ile Glu Gly Ser Tyr Gln Leu Glu Lys Val
            305                 310                 315

ctc ccc agt ctg ggg atc agt aac gtc ttc acc tcc cat gct gat ctg 1009
Leu Pro Ser Leu Gly Ile Ser Asn Val Phe Thr Ser His Ala Asp Leu
        320                 325                 330

tcc ggc atc agc aac cac tca aat atc cag gtg tct gag atg gtg cac 1057
Ser Gly Ile Ser Asn His Ser Asn Ile Gln Val Ser Glu Met Val His
    335                 340                 345

aaa gct gtg gtg gag gtg gac gag tcg gga acc aga gca gcg gca gcc 1105

Lys Ala Val Val Glu Val Asp Glu Ser Gly Thr Arg Ala Ala Ala Ala
350                 355                 360                 365

acg ggg aca ata ttc act ttc agg tcg gcc cgc ctg aac tct cag agg     1153
Thr Gly Thr Ile Phe Thr Phe Arg Ser Ala Arg Leu Asn Ser Gln Arg
                370                 375                 380

cta gtg ttc aac agg ccc ttt ctg atg ttc att gtg gat aac aac atc     1201
Leu Val Phe Asn Arg Pro Phe Leu Met Phe Ile Val Asp Asn Asn Ile
            385                 390                 395

ctc ttc ctt ggc aaa gtg aac cgc ccc gga tcc gac tac aag gac gac     1249
Leu Phe Leu Gly Lys Val Asn Arg Pro Gly Ser Asp Tyr Lys Asp Asp
        400                 405                 410

gat gac aag tga                                                     1261
Asp Asp Lys
    415

<210>  7
<211>  416
<212>  PRT
<213>  Artificial

<220>
<223>  Human PCI with Flag-tag

<220>
<221>  sig_peptide
<222>  (1)..(19)

<400>  7
Met Gln Leu Phe Leu Leu Leu Cys Leu Val Leu Leu Ser Pro Gln Gly
1               5                   10                  15

Ala Ser Leu His Arg His His Pro Arg Glu Met Lys Lys Arg Val Glu
            20                  25                  30

Asp Leu His Val Gly Ala Thr Val Ala Pro Ser Ser Arg Arg Asp Phe
        35                  40                  45

Thr Phe Asp Leu Tyr Arg Val Leu Ala Ser Ala Ala Pro Ser Gln Asn
    50                  55                  60

Ile Phe Phe Ser Pro Val Ser Ile Ser Met Ser Leu Ala Met Leu Ser
65                  70                  75                  80

Leu Gly Ala Gly Ser Ser Thr Lys Met Gln Ile Leu Glu Gly Leu Gly
                85                  90                  95

Leu Asn Leu Gln Lys Ser Ser Glu Glu Glu Leu His Arg Gly Phe Gln
            100                 105                 110

Gln Leu Leu Gln Glu Leu Asn Gln Pro Arg Asp Gly Phe Gln Leu Ser
        115                 120                 125

Leu Gly Asn Ala Leu Phe Thr Asp Leu Val Val Asp Leu Gln Asp Thr
    130                 135                 140

Phe Val Ser Ala Met Lys Thr Leu Tyr Leu Ala Asp Thr Phe Pro Thr
145                 150                 155                 160

Asn Phe Arg Asp Ser Ala Gly Ala Met Lys Gln Ile Asn Asp Tyr Val
                165                 170                 175

Ala Lys Gln Thr Lys Gly Lys Ile Val Asp Leu Leu Lys Asn Leu Asp
            180                 185                 190

Ser Asn Ala Val Val Ile Met Val Asn Tyr Ile Phe Phe Lys Ala Lys
        195                 200                 205

Trp Glu Thr Ser Phe Asn His Lys Gly Thr Gln Glu Gln Asp Phe Tyr
    210                 215                 220

Val Thr Ser Glu Thr Val Val Arg Val Pro Met Met Ser Arg Glu Asp
225                 230                 235                 240

Gln Tyr His Tyr Leu Leu Asp Arg Asn Leu Ser Cys Arg Val Val Gly
                245                 250                 255

Val Pro Tyr Gln Gly Asn Ala Thr Ala Leu Phe Ile Leu Pro Ser Glu
            260                 265                 270

Gly Lys Met Gln Gln Val Glu Asn Gly Leu Ser Glu Lys Thr Leu Arg
        275                 280                 285

Lys Trp Leu Lys Met Phe Lys Lys Arg Gln Leu Glu Leu Tyr Leu Pro
    290                 295                 300

Lys Phe Ser Ile Glu Gly Ser Tyr Gln Leu Glu Lys Val Leu Pro Ser
305                 310                 315                 320

Leu Gly Ile Ser Asn Val Phe Thr Ser His Ala Asp Leu Ser Gly Ile
                325                 330                 335

Ser Asn His Ser Asn Ile Gln Val Ser Glu Met Val His Lys Ala Val
            340                 345                 350

Val Glu Val Asp Glu Ser Gly Thr Arg Ala Ala Ala Ala Thr Gly Thr
        355                 360                 365

Ile Phe Thr Phe Arg Ser Ala Arg Leu Asn Ser Gln Arg Leu Val Phe
    370                 375                 380

Asn Arg Pro Phe Leu Met Phe Ile Val Asp Asn Asn Ile Leu Phe Leu
385                 390                 395                 400

Gly Lys Val Asn Arg Pro Gly Ser Asp Tyr Lys Asp Asp Asp Asp Lys
                405                 410                 415

<210> 8
<211> 119
<212> PRT
<213> Mus musculus

<400> 8
Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asp Ile Lys Asp Thr
            20                  25                  30

Phe Met His Trp Val Lys Gln Arg Pro Glu Gln Gly Leu Glu Trp Ile
        35                  40                  45

Gly Arg Ile Asp Tyr Val Asn Gly Asn Thr Lys Tyr Asp Pro Lys Phe
    50                  55                  60

Gln Gly Lys Ala Thr Ile Thr Gly Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Arg Gly Gly Tyr Asp Val Arg Glu Phe Ala Tyr Trp Gly Gln Gly
            100                 105                 110

Thr Leu Val Thr Val Ser Ala
        115

<210> 9

<211>  119
<212>  PRT
<213>  Mus musculus

<400>  9
Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asp Ile Lys Asp Thr
            20                  25                  30

Phe Met His Trp Val Lys Gln Arg Pro Glu Gln Gly Leu Glu Trp Ile
        35                  40                  45

Gly Arg Ile Asp Tyr Val Asn Gly Asn Thr Lys Tyr Asp Pro Lys Phe
    50                  55                  60

Gln Gly Lys Ala Thr Ile Thr Gly Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Arg Gly Gly Tyr Asp Val Arg Glu Phe Ala Tyr Trp Gly Gln Gly
            100                 105                 110

Thr Leu Val Thr Val Ser Ala
        115


<210>  10
<211>  119
<212>  PRT
<213>  Mus musculus

<400>  10

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asp Ile Arg Asp Thr
            20                  25                  30

Phe Met His Trp Val Lys Gln Arg Pro Glu Gln Gly Leu Glu Trp Ile
        35                  40                  45

Gly Arg Ile Asp Leu Val Asn Val Asn Thr Lys Tyr Asp Pro Asn Phe
    50                  55                  60

Gln Asp Arg Ala Thr Ile Thr Ala Asp Thr Ser Ser Asn Thr Ala Tyr
65                  70                  75                  80

Leu Gln Leu Thr Ser Leu Thr Ser Glu Asp Thr Ala Val Tyr Tyr Cys
                85                  90                  95

Ala Arg Gly Gly Tyr Asp Val Arg Glu Phe Ala Tyr Trp Gly Gln Gly
            100                 105                 110

Thr Leu Val Thr Val Ser Ala
        115

<210>  11
<211>  119
<212>  PRT
<213>  Mus musculus

<400>  11
Glu Val Gln Leu Gln Gln Ser Gly Ala Glu Leu Val Arg Pro Gly Ala
1               5                   10                  15

Leu Val Lys Leu Ser Cys Lys Ala Ser Gly Phe Asn Ile Lys Asp Tyr
            20                  25                  30

Tyr Ile His Trp Val Lys Gln Arg Pro Glu Gln Gly Leu Glu Trp Ile
            35                  40                  45

Gly Arg Ile Asp Leu Glu Lys Gly Asn Ile Ile Tyr Asp Pro Lys Phe
    50                  55                  60

Gln Gly Lys Asp Asn Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Arg Gly Gly Tyr Asp Val Pro Ser Phe Ala Tyr Trp Gly Gln Gly
            100                 105                 110

Thr Leu Val Thr Val Ser Ala
        115

<210>  12
<211>  119
<212>  PRT
<213>  Mus musculus

<400>  12
Glu Val Lys Leu Leu Glu Ser Gly Gly Gly Leu Val Gln Pro Gly Gly
1               5                   10                  15

Ser Leu Lys Leu Ser Cys Ala Ala Ser Gly Phe Asp Phe Ser Arg Tyr
            20                  25                  30

Trp Met Ser Trp Val Arg Gln Ala Pro Gly Lys Gly Leu Glu Trp Ile
            35                  40                  45

Gly Glu Ile Asn Pro Asp Ser Ser Thr Ile Asn Tyr Thr Pro Ser Leu

    50                  55                  60

Lys Asp Lys Phe Ile Ile Ser Arg Asp Asn Ala Lys Lys Thr Leu Tyr
65                  70                  75                  80

Leu Gln Met Asn Lys Val Arg Ser Glu Asp Thr Ala Leu Tyr Tyr Cys
                85                  90                  95

Ala Arg Phe Phe Tyr Tyr Gly Thr Pro Asp Tyr Trp Gly Gln Gly Thr
            100                 105                 110

Thr Leu Thr Val Ser Ser Ala
        115

<210> 13
<211> 119
<212> PRT
<213> Mus musculus

<400> 13
Glu Val Lys Leu Leu Glu Ser Gly Gly Gly Leu Val Gln Pro Gly Gly
1               5                   10                  15

Ser Leu Lys Phe Ser Cys Glu Ala Ser Gly Phe Asp Phe Ser Arg Tyr
            20                  25                  30

Trp Met Ser Trp Val Arg Gln Ala Pro Gly Lys Gly Leu Glu Trp Ile
        35                  40                  45

Gly Glu Ile Asn Pro Asp Ser Ser Thr Ile Thr Tyr Thr Ser Ser Leu
    50                  55                  60

Lys Asp Arg Phe Ile Ile Ser Arg Asp Asn Ala Lys Asn Thr Val Tyr
65                  70                  75                  80

Leu Gln Met Ser Lys Val Arg Ser Glu Asp Thr Ala Leu Tyr Tyr Cys
                85                  90                  95

Ala Arg Leu Phe Tyr Tyr Gly Thr Pro Asp Tyr Trp Gly Gln Gly Thr
            100                 105                 110

Thr Leu Thr Val Ser Ser Ala
        115

<210> 14
<211> 120
<212> PRT
<213> Mus musculus

<400> 14
Gln Val Gln Leu Gln Gln Ser Gly Ala Glu Leu Val Lys Pro Gly Ala
1               5                   10                  15

Ser Val Lys Met Ser Cys Lys Ala Phe Gly Tyr Thr Phe Thr Thr Tyr
            20                  25                  30

Pro Ile Glu Trp Met Lys Gln Asn His Gly Lys Ser Leu Glu Trp Ile
        35                  40                  45

Gly Lys Phe His Pro Asp Asn Asp Asp Thr Asn Tyr Asn Glu Lys Phe
    50                  55                  60

Lys Gly Lys Ala Lys Leu Thr Val Glu Lys Ser Ser Ser Thr Val Tyr
65                  70                  75                  80

Leu Glu Leu Ser Arg Leu Thr Ser Asp Asp Ser Ala Val Tyr Tyr Cys
                85                  90                  95

Ala Arg Gly His Asp Tyr Asp Tyr Gly Met Asp Tyr Trp Gly Gln Gly
            100                 105                 110

Thr Ser Val Thr Val Ser Ser Ala
        115                 120

<210>  15
<211>  106
<212>  PRT
<213>  Mus musculus

<400>  15
Gln Ile Val Leu Thr Gln Ser Pro Ala Ile Met Ser Ala Ser Pro Gly
1               5                   10                  15

Glu Lys Val Thr Ile Thr Cys Ser Ala Thr Ser Ser Leu Ile Tyr Met
            20                  25                  30

His Trp Phe Gln Gln Lys Pro Gly Ser Ser Pro Glu Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Ser Ser Tyr Pro Phe Thr
                85                  90                  95

Phe Gly Ser Gly Thr Lys Leu Glu Ile Lys
            100                 105

<210>  16
<211>  106
<212>  PRT

<213>  Mus musculus

<400>  16
Gln Ile Val Leu Thr Gln Ser Pro Ala Ile Met Ser Ala Ser Pro Gly
1               5                   10                  15

Glu Lys Val Thr Ile Thr Cys Ser Ala Thr Ser Ser Leu Ile Tyr Met
            20                  25                  30

His Trp Phe Gln Gln Lys Pro Gly Ser Ser Pro Glu Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Ser Ser Tyr Pro Phe Thr
                85                  90                  95

Phe Gly Ser Gly Thr Lys Leu Glu Ile Lys
            100                 105

<210>  17
<211>  106
<212>  PRT
<213>  Mus musculus

<400>  17
Gln Ile Val Leu Thr Gln Ser Pro Ala Ile Met Ser Ala Ser Pro Gly
1               5                   10                  15

Glu Lys Val Thr Ile Thr Cys Ser Ala Thr Ser Ser Leu Ile Tyr Met
            20                  25                  30

His Trp Phe Gln Gln Lys Pro Gly Thr Ser Pro Lys Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Ser Ser Tyr Pro Phe Thr
                85                  90                  95

Phe Gly Ser Gly Thr Lys Leu Glu Ile Lys
            100                 105

<210> 18
<211> 106
<212> PRT
<213> Mus musculus

<400> 18
Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Ser Tyr Met
            20                  25                  30

His Trp Phe Gln Gln Lys Pro Gly Thr Ser Pro Lys Leu Trp Ile Tyr
        35                  40                  45

Ser Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu

65                  70                  75                  80

Asp Ala Ala Thr Tyr Tyr Cys Gln Gln Arg Ser Ser Tyr Pro Phe Thr
                85                  90                  95

Phe Gly Ser Gly Thr Lys Leu Glu Ile Lys
            100                 105

<210>  19
<211>  108
<212>  PRT
<213>  Mus musculus

<400>  19
Asp Ile Val Met Thr Gln Ser His Lys Phe Met Ser Ala Ser Val Gly
1               5                   10                  15

Asp Arg Val Ser Ile Thr Cys Lys Ala Ser Gln Asp Val Ile Val Ala
            20                  25                  30

Val Ala Trp Tyr Gln Gln Lys Pro Gly Gln Ser Pro Glu Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Ser Pro Pro
                85                  90                  95

Trp Thr Phe Gly Gly Gly Thr Lys Leu Glu Ile Lys
            100                 105

<210>  20
<211>  108
<212>  PRT
<213>  Mus musculus

<400>  20
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ile Lys Ala
            20                  25                  30

Val Ala Trp Tyr Gln Gln Lys Pro Gly Gln Ser Pro Lys Leu Leu Ile
        35                  40                  45

Tyr Ser Thr Ser Tyr Arg Tyr Thr Gly Val Pro Asp Arg Phe Ser Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Ser Pro Pro
                85                  90                  95

Trp Thr Phe Gly Gly Gly Thr Lys Leu Glu Ile Lys
            100                 105


<210>  21
<211>  111
<212>  PRT
<213>  Mus musculus

<400>  21
Asp Ile Val Leu Thr Gln Ser Pro Ala Ser Leu Ala Val Ser Leu Gly

1               5                   10                  15

Gln Arg Ala Thr Ile Ser Cys Lys Ala Ser Gln Ser Val Asp Tyr Asp
            20                  25                  30

Gly Asp Ser Tyr Leu Asn Trp Tyr Gln Gln Lys Pro Gly Gln Pro Pro
        35                  40                  45

Lys Leu Leu Ile Tyr Gly Ala Ser Asn Leu Glu Ser Gly Thr Pro Ala
    50                  55                  60

Arg Phe Ser Gly Ser Gly Ser Gly Thr Asp Phe Thr Leu Asp Ile His
65                  70                  75                  80

Pro Val Glu Glu Glu Asp Ala Ala Thr Tyr Tyr Cys Gln Gln Ser Asn
                85                  90                  95

Glu Asp Pro Pro Thr Phe Gly Gly Gly Thr Lys Leu Glu Ile Thr
            100                 105                 110

<210>  22
<211>  5
<212>  PRT
<213>  Mus musculus

<400>  22
Asp Thr Phe Met His
1               5

<210>  23
<211>  5
<212>  PRT
<213>  Mus musculus

<400>  23
Asp Tyr Tyr Ile His
1               5

<210>  24
<211>  5
<212>  PRT
<213>  Mus musculus

<400>  24
Arg Tyr Trp Met Ser
1               5

<210>  25
<211>  5
<212>  PRT
<213>  Mus musculus

<400>  25
Thr Tyr Pro Ile Glu
1               5

<210>  26
<211>  17
<212>  PRT
<213>  Mus musculus

<400>  26
Arg Ile Asp Tyr Val Asn Gly Asn Thr Lys Tyr Asp Pro Lys Phe Gln
1               5                   10                  15

Gly

<210>  27
<211>  17
<212>  PRT
<213>  Mus musculus

<400>  27
Arg Ile Asp Leu Val Asn Val Asn Thr Lys Tyr Asp Pro Asn Phe Gln
1               5                   10                  15

Asp

<210>  28
<211>  17
<212>  PRT
<213>  Mus musculus

<400>  28
Arg Ile Asp Leu Glu Lys Gly Asn Ile Ile Tyr Asp Pro Lys Phe Gln
1               5                   10                  15

Gly

<210>  29
<211>  17
<212>  PRT
<213>  Mus musculus

<400>  29
Glu Ile Asn Pro Asp Ser Ser Thr Ile Asn Tyr Thr Pro Ser Leu Lys
1               5                   10                  15

Asp

<210> 30
<211> 17
<212> PRT
<213> Mus musculus

<400> 30
Glu Ile Asn Pro Asp Ser Ser Thr Ile Thr Tyr Thr Ser Ser Leu Lys
1               5                   10                  15

Asp

<210> 31
<211> 17
<212> PRT
<213> Mus musculus

<400> 31
Lys Phe His Pro Asp Asn Asp Asp Thr Asn Tyr Asn Glu Lys Phe Lys
1               5                   10                  15

Gly

<210> 32
<211> 10
<212> PRT
<213> Mus musculus

<400> 32
Gly Gly Tyr Asp Val Arg Glu Phe Ala Tyr
1               5                   10

<210> 33
<211> 10
<212> PRT
<213> Mus musculus

<400> 33
Gly Gly Tyr Asp Val Pro Ser Phe Ala Tyr
1               5                   10

<210> 34
<211> 9
<212> PRT
<213> Mus musculus

<400> 34
Phe Phe Tyr Tyr Gly Thr Pro Asp Tyr
1               5

<210> 35
<211> 9
<212> PRT
<213> Mus musculus

<400> 35
Leu Phe Tyr Tyr Gly Thr Pro Asp Tyr
1               5

<210> 36

<211>  10
<212>  PRT
<213>  Mus musculus

<400>  36
Gly His Asp Tyr Asp Tyr Gly Met Asp Tyr
1               5                   10

<210>  37
<211>  10
<212>  PRT
<213>  Mus musculus

<400>  37
Ser Ala Thr Ser Ser Leu Ile Tyr Met His
1               5                   10

<210>  38
<211>  10
<212>  PRT
<213>  Mus musculus

<400>  38
Ser Ala Ser Ser Ser Val Ser Tyr Met His
1               5                   10

<210>  39
<211>  11
<212>  PRT
<213>  Mus musculus

<400>  39
Lys Ala Ser Gln Asp Val Ile Val Ala Val Ala

1               5                   10

<210>  40
<211>  11
<212>  PRT
<213>  Mus musculus

<400>  40
Lys Ala Ser Gln Asp Val Ile Lys Ala Val Ala
1               5                   10

<210>  41
<211>  15
<212>  PRT
<213>  Mus musculus

<400>  41
Lys Ala Ser Gln Ser Val Asp Tyr Asp Gly Asp Ser Tyr Leu Asn
1               5                   10                  15

<210>  42
<211>  11
<212>  PRT
<213>  Mus musculus

<400>  42
Ser Thr Ser Asn Leu Ala Ser Gly Val Pro Ala
1               5                   10

<210>  43
<211>  11
<212>  PRT

<213>  Mus musculus

<400>  43
Ser Ala Ser Tyr Arg Tyr Thr Gly Val Pro Asp
1               5                   10

<210>  44
<211>  11
<212>  PRT
<213>  Mus musculus

<400>  44
Ser Thr Ser Tyr Arg Tyr Thr Gly Val Pro Asp
1               5                   10

<210>  45
<211>  11
<212>  PRT
<213>  Mus musculus

<400>  45
Gly Ala Ser Asn Leu Glu Ser Gly Thr Pro Ala
1               5                   10

<210>  46
<211>  7
<212>  PRT
<213>  Mus musculus

<400>  46
Arg Ser Ser Tyr Pro Phe Thr
1               5

<210>  47
<211>  8
<212>  PRT
<213>  Mus musculus

<400>  47
His Tyr Ser Ser Pro Pro Trp Thr
1               5

<210>  48
<211>  7
<212>  PRT
<213>  Mus musculus

<400>  48
Ser Asn Glu Asp Pro Pro Thr
1               5

<210>  49
<211>  5
<212>  PRT
<213>  Artificial

<220>
<223>  Heavy chain CDR1

<220>
<221>  misc_feature
<222>  (2)..(2)
<223>  "Xaa" in position 2 represents "Thr" or "Tyr"

<220>
<221>  misc_feature

<222>  (3)..(3)
<223>  "Xaa" in position 3 represents "Phe" or "Tyr"

<220>
<221>  misc_feature
<222>  (4)..(4)
<223>  "Xaa" in position 4 represents "Met" or "Ile"

<400>  49
Asp Xaa Xaa Xaa His
1               5

<210>  50
<211>  17
<212>  PRT
<213>  Artificial

<220>
<223>  Heavy chain CDR2

<220>
<221>  misc_feature
<222>  (4)..(4)
<223>  "Xaa" in position 4 represents "Tyr" or "Leu"

<220>
<221>  misc_feature
<222>  (5)..(5)
<223>  "Xaa" in position 5 represents "Val" or "Glu"

<220>
<221>  misc_feature
<222>  (6)..(6)
<223>  "Xaa" in position 6 represents "Asn" or "Lys"

<220>
<221> misc_feature
<222> (7)..(7)
<223> "Xaa" in position 7 represents "Gly" or "Val"

<220>
<221> misc_feature
<222> (9)..(9)
<223> "Xaa" in position 9 represents "Thr" or "Ile"

<220>
<221> misc_feature
<222> (10)..(10)
<223> "Xaa" in position 10 represents "Lys" or "Ile"

<220>
<221> misc_feature
<222> (14)..(14)
<223> "Xaa" in position 14 represents "Lys" or "Asn"

<220>
<221> misc_feature
<222> (17)..(17)
<223> "Xaa" in position 17 represents "Gly" or "Asp"

<400> 50
Arg Ile Asp Xaa Xaa Xaa Xaa Asn Xaa Xaa Tyr Asp Pro Xaa Phe Gln
1               5                   10                  15

Xaa

<210> 51
<211> 10
<212> PRT

<213>  Artificial

<220>
<223>  Heavy chain CDR3

<220>
<221>  misc_feature
<222>  (6)..(6)
<223>  "Xaa" in position 6 represents "Arg" or "Pro"

<220>
<221>  misc_feature
<222>  (7)..(7)
<223>  "Xaa" in position 7 represents "Glu" or "Ser"

<400>  51
Gly Gly Tyr Asp Val Xaa Xaa Phe Ala Tyr
1               5                   10

<210>  52
<211>  5
<212>  PRT
<213>  Artificial

<220>
<223>  Heavy chain CDR1

<400>  52
Arg Tyr Trp Met Ser
1               5

<210>  53
<211>  17
<212>  PRT

<213>  Artificial

<220>
<223>  Heavy chain CDR2

<220>
<221>  misc_feature
<222>  (10)..(10)
<223>  "Xaa" in position 10 represents "Asn" or "Thr"

<220>
<221>  misc_feature
<222>  (13)..(13)
<223>  "Xaa" in position 13 represents "Pro" or "Ser"

<400>  53
Glu Ile Asn Pro Asp Ser Ser Thr Ile Xaa Tyr Thr Xaa Ser Leu Lys
1               5                   10                  15

Asp

<210>  54
<211>  9
<212>  PRT
<213>  Artificial

<220>
<223>  Heavy chain CDR3

<220>
<221>  misc_feature
<222>  (1)..(1)
<223>  "Xaa" in position 1 represents "Phe" or "Leu"

<400>  54
Xaa Phe Tyr Tyr Gly Thr Pro Asp Tyr
1               5

<210>  55
<211>  10
<212>  PRT
<213>  Artificial

<220>
<223>  Light chain CDR1

<220>
<221>  misc_feature
<222>  (3)..(3)
<223>  "Xaa" in position 3 represents "Thr" or "Ser"

<220>
<221>  misc_feature
<222>  (6)..(6)
<223>  "Xaa" in position 6 represents "Leu" or "Val"

<220>
<221>  misc_feature
<222>  (7)..(7)
<223>  "Xaa" in position 7 represents "Ile" or "Ser"

<400>  55
Ser Ala Xaa Ser Ser Xaa Xaa Tyr Met His
1               5                   10

<210>  56
<211>  11
<212>  PRT

<213>  Artificial

<220>
<223>  Light chain CDR2

<400>  56
Ser Thr Ser Asn Leu Ala Ser Gly Val Pro Ala
1               5                   10

<210>  57
<211>  7
<212>  PRT
<213>  Artificial

<220>
<223>  Light chain CDR3

<400>  57
Arg Ser Ser Tyr Pro Phe Thr
1               5

<210>  58
<211>  11
<212>  PRT
<213>  Artificial

<220>
<223>  Light chain CDR1

<220>
<221>  misc_feature
<222>  (8)..(8)
<223>  "Xaa" in position 8 represents "Val" or "Lys"

<400> 58
Lys Ala Ser Gln Asp Val Ile Xaa Ala Val Ala
1               5                   10

<210> 59
<211> 11
<212> PRT
<213> Artificial

<220>
<223> Light chain CDR2

<220>
<221> misc_feature
<222> (2)..(2)
<223> "Xaa" in position 2 represents "Ala" or "Thr"

<400> 59
Ser Xaa Ser Tyr Arg Tyr Thr Gly Val Pro Asp
1               5                   10

<210> 60
<211> 8
<212> PRT
<213> Artificial

<220>
<223> Light chain CDR3

<400> 60
His Tyr Ser Ser Pro Pro Trp Thr
1               5